# EUROPEAN PATENT APPLICATION

(11) **EP 1 505 152 A1**
(43) Date of publication of application: **09.02.2005**
(21) Application number: 03719214.3
(22) Date of filing: 25.04.2003
(51) Int. Cl.: C12N 15/11, C12N 5/10, C12N 1/19, A01K 67/027, C12Q 1/02, C12Q 1/68, A61K 31/7088, A61K 48/00

(54) **EXPRESSION SYSTEMS FOR STEM LOOP RNA MOLECULE HAVING RNAi EFFECT**

(30) Priority: 26.04.2002 JP 2002127089; 10.01.2003 JP 2003004706; 27.02.2003 US 449860 P
(71) Applicant: National Institute of Advanced Industrial Science and Technology, Tokyo 100-8921 (JP)
(72) Inventor: TAIRA, Kazunari, Tsukuba-shi, Ibaraki 305-0046 (JP); KAWASAKI, Hiroaki, Minato-ku, Tokyo 107-0052 (JP)
(74) Representative: Grünecker, Kinkeldey, Stockmair & Schwanhäusser Anwaltssozietät
(86) International application number: PCT/JP2003/005373
(87) International publication number: WO 2003/091433

(57) **Abstract**

The present inventors discovered that an RNA molecule can be efficiently transferred into cytoplasm and exert RNAi effects, by producing a stem loop RNA molecule from the DNA that encodes this RNA molecule, with the use of a tRNA promoter. Also, the RNAi effects can be exerted effectively, by introducing a cytoplasm translocation signal sequence into the DNA that encodes a stem loop RNA molecule. Moreover, the RNAi effects can be exerted effectively by transferring a transcriptional product into the cytoplasm, using a pol II-type promoter. In this case, cytotoxicity can be reduced by co-expressing a Dicer gene. Furthermore, an effective dsRNA can be constructed by treating a dsRNA or a stem loop RNA molecule with Dicer protein. Knockout cells lacking a gene of interest can be conveniently constructed, using these stem-loop RNA molecule expression systems.

## Description

### Technical Field

The present invention relates to expression systems for RNA molecules which can suppress target gene expression, and methods for producing knockdown cells using these systems.

### Background Art

RNA interference (hereinafter abbreviated as "RNAi") is a phenomenon, in which the mRNA degradation of a target gene is induced and thus the target gene expression is suppressed, by introducing into cells or such, a double-stranded RNA (hereinafter abbreviated as "dsRNA") which comprises a sense RNA comprising a sequence homologous to a target gene mRNA, and an antisense RNA comprising the complementary sequence of the sense RNA. As described above, since RNAi can be used to suppress target gene expression, RNAi has drawn attention as a simpler gene knockout method, alternative to the more complicated and less efficient gene disruption methods using homologous recombination, or as a method applicable to gene therapy. The RNAi phenomenon described above was originally found in Nematode (Fire, A. et al., "Potent and specific genetic interference by double-stranded RNA in Caenorhabditis elegans." Nature, 391, 806-811, 1998) . Currently, the phenomenon can be observed not only in Nematode but also in various organisms, including plants, Nemathelminthes, fruitflies, and protozoa (Fire, A., "RNA-triggered gene silencing." Trends Genet., 15, 358-363, 1999; Sharp, P. A., "RNA interference 2001." Genes Dev. , 15, 485-490, 2001; Sharp, P. A., "RNA interference 2001." Genes Dev., 15, 485-490, 2001; Hammond, S. M., Caudy, A. A., & Hannon, G. J., "Post-transcriptional gene silencing by double-stranded RNA." Nature Rev. Genet., 2, 110-119, 2001; Zamore, P. D., "RNA interference: listening to the sound of silence." Nat. Struct. Biol. , 8, 746-750, 2001). It has been confirmed that indeed, target gene expressions are suppressed upon introduction of foreign dsRNAs in the above-mentioned organisms. RNAi is also being used as a method to create knockout animals and plants.

dsRNAs are known to target mRNAs *in vitr*o for cleavage in lysates of early *Drosophila* embryos or extracts of cultured *Drosophil*a S2 cells (Tuschl, T. et al. , Genes Dev., 13, 3191-3197, 1999; Hammond, S. M. et al., Nature, 404, 293-296, 2000; Zamore P. et al., Cell, 101, 25-33, 2000). Reactions of RNAi *in vitro* require ATP (Hammond, S. M. et al, Nature, 404, 203-296, 2000; Zamore, P. et al., Cell, 101, 25-33, 2000).

Recent studies using synthetic RNA duplexes demonstrated that siRNA duplexes cleave their respective target RNAs (Elbashir, S. M. et al., Genes Dev., 15, 188-200, 2001). It became apparent that 2-or 3-nt 3'-overhanging ends within the siRNA duplex are required for efficient target cleavage (Elbashir, S. M. et al., Genes Dev., 15, 188-200, 2001). Such 3' overhangs are characteristic of the products of an RNase III cleavage reaction, and in cultured *Drosophila* S2 cells, cleavage of the dsRNA into siRNAs requires a multidomain RNase III enzyme, known as Dicer (Bernstein, E. et al., Nature, 409, 363-366, 2001). Subsequently, siRNAs seem to associate with a multicomponent nuclease identified in *Drosophila,* called RISC, and guide this enzyme for sequence-specific degradation of mRNAs (Hammond, S. M. et al., Nature, 404, 293-296, 2000; Bernstein, E. et al. , Nature 409, 363-366, 2001, Hammond, S. M. et al., Science 293, 1146-1150, 2001).

RNAi provides methods to inactivate genes of interest, and thus serves as a powerful tool for studying gene function in *C*. *elegans, Drosophila* and plants. Specific inhibition of gene expression can also be achieved by stable expression and inducible expression of dsRNAs in animals and plants (Hammond, S. M. , Caudy, A. A. , & Hannon, G. J., "Post-transcriptional gene silencing by double-stranded RNA." Nature Rev. Genet., 2, 110-119, 2001; Kennerdell & Carthew, Nature Biotechnol, 18, 896-898, 2000; Tavernarakis, N. et al., Nature Genetics, 24, 180-183, 2001). Although inactivation of genes using dsRNA was successful in mouse embryonal carcinoma (EC) cells and embryonic stem (ES) cells (Billy, E. et al., PNAS, 98, 14428-14433, 2001; Paddison, P. et al. , PNAS, 99, 1443-1448, 2002) , RNAi induction using long dsRNAs in cultured mammalian cells has generally been less successful. These failures are readily explained by the action of two latent enzymes forming part of the interferon (IFN) defense pathways, which are activated by long dsRNAs (>30 base pair) (Stark, G. R. et al., Annu. Rev. Biochem., 67, 227-264, 1998). One of them is 2'-5'-oligoadenylate (2-5A) synthase, which is activated by dsRNA to increase synthesis of 2-5A, required for activation of the nonsequence-specific RNase, called RNase L (Silverman, R. H., in "Ribonucleases: Structures and Functions" D'Alessio, G. & Riordan J. F. eds., Academic, New York, pp.515-551, 1997). The other is protein kinase PKR, the active form of which phosphorylates the translation factor eukaryotic initiation factor 2 (eIF2), causing an overall inhibition of protein synthesis and cell death (Clemens, M. J. and Elia, A., J. Interferon Cytokine Res., 17, 503-524, 1997).

21-nucleotide siRNA duplexes were recently reported to specifically suppress expression of endogenous genes in several mammalian cells (Elbashir, S. M. et al. , Nature, 411, 494-498, 2001). In this case, 21-nucleotides siRNA duplexes are protected from the IFN defense system. These findings suggested that RNAi or RNAi-related system exists in mammals. Indeed, some of mammalian homologs of RNAi-associated proteins such as *red-1, mut-7*, and Dicer were identified (Bernstein, E. et al., Nature, 409, 363-366, 2001; Tabara, H. et al., Cell, 99, 123-132, 1999; Ketting, R. F. et al., Cell, 99, 133-141, 1999). However, characteristics and mechanisms of RNAi in mammalian somatic cells are still unclear.

The use of RNAi to suppress gene expression also shows promise in functional analysis and gene therapy. Nearly all primary sequences of genes have been revealed, and systematic and efficient functional gene screening methods are being developed to quickly unravel gene functions. Identification of novel functional genes can be accelerated by using RNAi to suppress expression of genes of interest, and using phenotypic alterations of these cells or animals as indicators to systematically search for functional genes.

### Disclosure of the Invention

The present invention was achieved in view of the above. An objective of the present invention is to provide novel RNA expression systems, in which expression of target genes can be suppressed, and methods for producing knockdown cells using such systems.

The present inventors performed exhaustive studies to solve the above-described issues. The inventors examined the cellular sites where RNAi effects take place in mammalian cells. First, the inventors constructed two types of dsRNA expression vectors, "tRNA-dsRNA" and "U6-dsRNA", which are regulated by tRNA^{Val} and U6 promoters, respectively. It has been reported that transcripts generated by U6 promoter remain in the nucleus, whereas transcripts generated by TRNA^{Val} promoter are efficiently translocated from the nucleus to cytoplasm.

Experimental results showed that tRNA-dsRNA transcripts were localized in the cytoplasm and efficiently processed by ribonuclease III complex. In addition, tRNA-dsRNA directed against mutant k-ras effectively cleaved target mRNAs both *in vitro* and *in vivo*, in other words indicating that RNAi effects have taken place. In contrast, U6-dsRNA did not affect the expression of normal k-ras in HeLa cells. Thus, these results suggest that in mammalian cells, RNAi is a cytoplasmic event. Furthermore, the present inventors revealed that RNAi effects can effectively suppress gene expression in yeasts.

The dsRNAs used in the above-described experiments are thought to form stem-loop structures in cells. Thus, the above-described experimental results suggest that RNAi effects are produced when RNA molecules with a stem-loop structure (stem-loop RNA molecules) are transported into the cytoplasm.

An RNA molecule forming the stem-loop structure is advantageous in that it can be transcribed as a single transcript from DNA.

The stem-loop RNA molecules expressed by U6 promoter are normally localized in the nucleus. In contrast, the stem-loop RNA molecules comprising microRNA loop sequences, designed by the present inventors, were transported into the cytoplasm and proved to be effective in producing RNAi effects. However, since transport of microRNAs into cytoplasm is essential for cellular maintenance, the U6-stem-loop RNA molecules which comprise microRNA loop sequences may act in a dominant-negative manner, and thus render cytotoxicity. In contrast, stem-loop RNA molecules developed by the present inventors, which are expressed by tRNA promoters, are expected to be free of cytotoxicity because they are transported into the cytoplasm without using the microRNA transport system.

As described above, the present inventors used tRNA promoters to produce stem-loop RNA molecules from DNAs that encode those RNA molecules, and succeeded in efficiently transporting those RNA molecules into the cytoplasm, producing RNAi effects, thereby completing the present invention.

The present inventors also performed the experiments below, based on the inventors' present finding that translocation of the above-described RNA molecules into the cytoplasm produces RNAi effects. Specifically, the inventors prepared recombinant human Dicer (hDicer) , and processed long dsRNA using the recombinant hDicer to yield siRNAs of 20 to 25 nt. The inventors examined RNAi effects by introducing these siRNAs directly into the cytoplasm. When introduced directly into the cytoplasm, siRNAs (diced-siRNAs) produced by treating long dsRNA substrates with the above-described hDicer, were found to effectively suppress target gene expression. The generation of RNAi effects by introducing recombinant hDicer-treated RNAs directly into cells, was achieved, for the first time, by the present inventors. It was also the present inventors who, again for the first time, generated recombinant hDicer proteins that possess activities of producing siRNAs.

Using the stem-loop RNA molecule expression system developed by the present inventors, knockout cells with disrupted genes of interest can be easily prepared. The present invention is highly anticipated to be a powerful tool for studying RNAi mechanisms and other gene functions in mammalian cells, and also as a potentially useful method for treating diseases.

The present invention relates to novel expression systems for RNA molecules, which can suppress target gene expressions, and methods for preparing knockdown cells using these systems. More specifically, the invention provides:
[1] a DNA encoding a stem-loop RNA molecule having an RNAi effect within a cell, wherein said DNA comprises (i) a cytoplasmic translocation signal sequence, and (ii) a structure in which (a) a sense coding DNA that encodes a sense RNA of any one of the regions of a target gene mRNA, and (b) a sequence complementary to said sense coding DNA are linked in opposite directions via a spacer region, and is operably linked to a promoter;
[2] the DNA according to [1], comprising said cytoplasmic translocation signal sequence within the spacer region;
[3] the DNA according to [1] or [2], wherein said promoter is a tRNA promoter, a pol II-type promoter, a pol III-type promoter, or a tetracycline-induced promoter;
[4] the DNA according to [3], wherein said tetracycline-induced promoter is tetracycline-induced tRNA promoter;
[5] the DNA according to [1] or [2], wherein said promoter is NMT1 promoter or GAL1 promoter;
[6] a DNA encoding a stem-loop RNA molecule having an RNAi effect within a cell, wherein said DNA comprises a structure in which (a) a sense coding DNA that encodes a sense RNA of any one of the regions of a target gene mRNA; and (b) a sequence complementary to said sense coding DNA are linked in opposite directions via a spacer region, and is operably linked to a promoter;
[7] the DNA according to [3], [4], or [6], wherein said tRNA promoter is TRNA ^{VAL} promoter;
[8] the DNA according to any one of [1] to [4] , [6] , and [7] , wherein the length of the sense coding DNA is 10 to 35 bp;
[9] the DNA according to any one of [1] to [4], [6), and [7], wherein the length of the sense coding DNA is 26 to 30 bp;
[10] the DNA according to any one of [1] to [4], [6], and [7] , wherein the length of the sense coding DNA is 31 to 35 bp;
[11] the DNA according to [5] , wherein the length of the sense coding DNA is 10 to 5000 bp;
[12] the DNA according to any one of [1] to [11] , wherein the length of the spacer region is 1 to 10000 nucleotides;
[13] the DNA according to any one of [1] to [11] , wherein the length of the spacer region is 1 to 100 nucleotides;
[14] the DNA according to any one of [1] to [4], [6] to [10], [12] , and [13] , wherein the stem region of the stem-loop RNA molecule to be expressed is 10 to 35 bp in length;
[15] the DNA according to any one of [1] to [4], [6] to [10], [12] and [13], wherein the stem region of the stem-loop RNA molecule to be expressed is 26 to 30 bp in length;
[16] the DNA according to any one of [1] to [4], [6] to [10], [12] and [13], wherein the stem region of the stem-loop RNA molecule to be expressed is 31 to 35 bp in length;
[17] the DNA according to any one of [1] to [16], wherein the stem-loop RNA molecule to be expressed is constructed to comprise a mismatch or bulge in the stem region;
[18] the DNA according to [17], wherein the sense strand is constructed to comprise a mismatch having one to six G-U nucleotides for every 20 base pairs;
[19] the DNA according to any one of [1] to [18], wherein the stem-loop RNA molecule to be expressed is constructed to comprise a mismatch or bulge of one to ten nucleotides for every 20 base pairs in the stem region;
[20] the DNA according to [19], wherein said mismatch has one to six G-U nucleotides for every 20 base pairs in the sense strand;
[21] the DNA according to [5] or [11], wherein the stem-loop RNA molecule to be expressed is constructed to comprise a mismatch or bulge of 1 to 100 nucleotides for every 300 base pairs in the stem region;
[22] the DNA according to any one of [1] to [21] , wherein said cell is a mammalian cell or yeast cell;
[23] a stem-loop RNA molecule having an RNAi effect within a cell, wherein said RNA is a transcription product of a DNA according to any one of [1] to [22];
[24] a vector that comprises a DNA according to any one of [1] to [22];
[25] a cell that carries a DNA according to any one of [1] to [22], or the vector according to [24];
[26] the cell according to [25], wherein said cell is a mammalian cell or yeast cell;
[27] a composition comprising a DNA according to any one of [1] to [22], or the vector according to [24];
[28] a method for producing cells in which the expression of a target gene is suppressed, wherein said method comprises the steps of introducing a DNA according to any one of [1] to [22], or the vector according to [24] into cells, and selecting cells carrying said DNA or vector;
[29] a vector comprising a DNA encoding a stem-loop random RNA molecule within a cell, wherein said DNA comprises (i) a cytoplasmic translocation signal sequence, and (ii) a structure in which (a) a sense coding DNA that encodes a sense RNA of any one of the regions of a target gene mRNA, and (b) a sequence complementary to said sense coding DNA are linked in opposite directions via a spacer region, and is operably linked to a tRNA promoter;
[30] an individual organism carrying a DNA according to any one of [1] to [22], or the vector according to [24] or [29];
[31] the individual organism according to [30], which is a non-human target gene-knockout animal;
[32] the individual organism according to [30] or [31], wherein said organism is selected from the group consisting of a mouse, rat, rabbit, cow, horse, pig, sheep, monkey, and chimpanzee;
[33] the vector according to [29], wherein the stem region of the stem-loop random RNA molecule to be expressed is 10 to 35 bp in length;
[34] a method of screening for a functional gene, wherein said method comprises the steps of:
   (a) introducing the vector according to [29] or [33] into cells,
   (b) selecting cells carrying the vector, and
   (c) analyzing the phenotype of said selected cells;
[35] the method of screening for a functional gene according to [34], which further comprises the step of screening for the functional gene based on the random sequences of a vector sequence, within a cell that is found to have altered phenotypes by phenotypic analysis;
[36] an siRNA expression system that comprises:
   (a) A DNA that encodes a stem-loop RNA molecule having an RNAi effect within a cell, wherein said DNA comprises a structure in which (i) a sense coding DNA that encodes a sense RNA of any one of the regions of a target gene mRNA, and (ii) a sequence complementary to said sense coding DNA are linked in opposite directions via a spacer region, and is operably linked to a promoter, and
   (b) a DNA comprising a structure, in which a DNA encoding a polypeptide region having the Dicer activity of a Dicer protein, and is operably linked to a promoter;
[37] the siRNA expression system according to [36], wherein said promoter according to (a) is a tRNA promoter or Pol III-type promoter;
[38] the siRNA expression system according to [36], wherein said promoter according to (a) is NMT1 promoter, GAL1 promoter, or Pol II-type promoter;
[39] the siRNA expression system according to [38], wherein the stem-loop RNA molecule to be expressed comprises a mismatch or bulge of 1 to 100 nucleotides for every 300 base pairs in the stem region;
[40] the siRNA expression system according to [36], wherein the promoter according to (b) is Pol II-type promoter;
[41] the siRNA expression system according to any one of [36] to [40], wherein said cell is a mammalian cell or yeast cell;
[42] the siRNA expression system according to any one of [36] to [41], wherein the length of the dsRNA resulting from the pairing of sense RNA with antisense RNA in any one of the regions of the target gene mRNA, or the length of the stem region in the stem-loop RNA molecule that has said dsRNA as the stem, is at least 30 base pairs or more;
[43] the siRNA expression system according to any one of [36] to [41], which comprises a mismatch or bulge of 1 to 100 nucleotides for every 300 base pairs in the dsRNA resulting from the pairing of sense RNA with antisense RNA in any one of the regions of the target gene mRNA, or in the sense strand within the stem region of the stem-loop RNA molecule that has said dsRNA as the stem;
[44] an siRNA expression vector containing the DNAs of both (a) and (b) of [36];
[45] a method for suppressing the expression of a target gene, wherein said method comprises expressing both a stem-loop RNA molecule and a polypeptide having Dicer activity, using the siRNA expression system according to any one of [36] to [43], or the expression vector according to [44];
[46] a method for suppressing the expression of a target gene wherein said method comprises the steps of:
   (a) treating a dsRNA that results from the pairing of sense RNA with antisense RNA in any one of the regions of the target gene mRNA, or the stem-loop RNA molecule having said dsRNA as the stem, with a polypeptide having Dicer activity, and
   (b) introducing the dsRNA produced in the above-mentioned step (a) into a cell comprising the target gene;
[47] the method according to [46], wherein said polypeptide having Dicer activity is a polypeptide comprising the amino acid sequence of positions 1066 to 1924 of Dicer protein;
[48] the method according to [46], wherein said polypeptide having Dicer activity is a polypeptide comprising the amino acid sequence of positions 1268 to 1924 of Dicer protein;
[49] the method according to [46], wherein said polypeptide having Dicer activity is a polypeptide comprising the amino acid sequence of positions 1296 to 1924 of Dicer protein;
[50] the method according to [46], wherein the polypeptide having Dicer activity is the full length Dicer protein;
[51] the method according to any one of [45] to [50], wherein said cell is a mammalian cell or yeast cell;
[52] the method according to [46], wherein said polypeptide having Dicer activity is a polypeptide according to any one of [47] to [50] expressed in *E. coli;*
[53] the method according to [46], wherein said polypeptide having Dicer activity is a polypeptide according to any one of [44] to [47] expressed in insect cells;
[54] the method according to any one of [45] to [53], wherein said dsRNA resulting from the pairing of sense RNA with antisense RNA in any one of the regions of the target gene mRNA, or the stem region of the stem-loop RNA molecule that has said dsRNA as the stem, has a length of at least 30 base pairs or more;
[55] the method according to any one of [45] to [53], comprising a mismatch or bulge of 1 to 100 nucleotides for every 300 base pairs in the dsRNA that results from the pairing of sense RNA with antisense RNA in any one of the regions of the target gene mRNA, or in the sense strand within the stem region of the stem-loop RNA molecule that has said dsRNA as the stem.

The present inventors discovered that stem-loop RNA molecules exhibit RNAi effects when translocated into the cytoplasm. The present inventors then demonstrated that, by linking tRNA promoters to DNAs that encode the stem-loop RNAs, transcripts transcribed by the promoters were efficiently transported from the nucleus to the cytoplasm, exhibiting RNAi effects. The term "stem loop", also called "hairpin loop", refers to a structure that comprises a double-stranded portion (stem), formed by hydrogen bonding between inverted repeat sequences in a single-stranded RNA molecule, and a loop portion sandwiched in between. Stem-loop RNA molecules can be thought of as molecules, in which the stem region comprises a double-stranded RNA structure, and that are processed in the cytoplasm by ribonuclease III complex (RISC) containing Dicer to produce short interfering RNA (siRNAs) that exhibit RNAi effects. Typical siRNAs are double-stranded RNA molecules of about 20 bp that exhibit RNAi effects. Dicer and RISC are assumed to be localized in the cytoplasm, thus, translocation of stem-loop RNA molecules into the cytoplasm is essential for producing the RNAi effects.

The present invention provides DNAs encoding stem-loop RNA molecules which exhibit RNAi effects in cells. Specifically, the present invention relates to DNAs encoding stem-loop RNA molecules that exhibit RNAi effects in cells, and these DNAs are characterized by the efficient translocation of their expression products into the cytoplasm.

In a preferred embodiment, the above-described DNAs of the present invention are characterized by their structure, in which a sense (or antisense) coding DNA that encodes a sense (or antisense) RNA of any regions of a target gene mRNA, and its complementary sequence are linked in opposite directions via a spacer region, and functionally linked to a promoter.

In the present invention, the term "target gene" refers to a gene whose expression is suppressed by RNAi effects of the stem-loop RNA molecules of this invention, and any gene may be selected as a target gene. A preferable target gene can be selected from, for example, genes that have identified sequences, but yet-to-be interpreted functions, or genes whose expressions are thought to trigger diseases. Genes with unknown genomic sequences can also be selected as the target gene, as long as a partial sequence of their mRNAs of at least 15 nucleotides or more is known. Thus, a gene can also be selected as the target gene in the present invention, when only partial sequences of its mRNA, such as Expressed Sequence Tags (ESTs), and not its full length sequence, are known.

The above-mentioned phrase "in opposite directions" means that one sequence is positioned in the opposition direction of the other sequence. In other words, the above-described DNAs of the present invention have an inverted repeat sequence structure, formed by sense RNA-encoding DNA sequences with a spacer region sandwiched in between. Specifically, for example, when a (double-stranded) DNA sequence encoding a sense RNA consists of: 
AGTC (sense strand)
::::
TCAG (antisense strand)
 The structure, in which an above-described DNA sequence encoding the sense RNA forms an inverted repeat sequence, comprising a spacer region in between is represented as: 
AGTC-SSS-GACT
::::     ::::
TCAG-SSS-CTGA
 (where "S" indicated above refers to an arbitrary nucleotide in the spacer region and ":" refers to a hydrogen bond).

As described above, the DNAs of the present invention have a structure in which a sense coding DNA that encodes a sense RNA of any regions of a target gene mRNA and its complementary sequence are linked in opposite directions via a spacer region. In other words, the "complementary sequence" is equivalent to "a DNA encoding an antisense RNA that corresponds to 'any regions of a target gene MRNA' " descried above. In other words, the DNAs of the present invention can also have a structure, in which an antisense coding DNA that encodes an antisense RNA of any regions of a target gene mRNA, and its complementary sequence are linked in opposite directions via a spacer region.

The above-described DNAs of the present invention can be efficiently transported into the cytoplasm, for example, by incorporating a cytoplasmic translocation signal sequence into the DNAs described above. Specifically, in a preferred embodiment, the DNAs of the present invention are said DNAs that comprise a cytoplasmic translocation signal sequence. There is no limitation as to the positions of a cytoplasmic translocation signal sequence, but the sequence is placed preferably within a spacer region. The cytoplasmic translocation signal sequences of the present invention can be known cytoplasmic translocation signals, or DNA sequences that can be specifically bound by a protein capable of translocating into the cytoplasm. An example of such cytoplasmic translocation signal sequences is the miR23 loop motif (sequence: 5'-CUUCCUGUCA-3') (Kawasaki, H., Taira, K., "Short hairpin type of dsRNAs that are controlled by tRNA(Val) promoter significantly induce RNAi-mediated gene silencing in the cytoplasm of human cells." Nucleic Acids Res., 31, 700-707, 2003).

The "promoter" in the above-described DNAs of the present invention can be a tRNA promoter, or a promoter for the polII or polIII system to be described below. A tetracycline-induced promoter can also be preferably used. The DNAs of the present invention may be expressed at a desired timing by using this kind of inducible promoter. Such tetracycline-induced promoters include, for example, tetracycline-inducible U6 promoter (Ohkawa, J., & Taira, K. , "Control of the functional activity of an antisense RNA by a tetracycline-responsive derivative of the human U6 snRNA promoter." Hum Gene Ther., 11, 577-585, 2000). Examples of the tetracycline-induced promoter include TetO7-comprising promoters. One may refer to Examples described below for the uses of tetracycline-induced promoters. A preferable promoter of the present invention is a tetracycline-induced tRNA promoter. Additional preferable promoters of the present invention include, for example, NMT1 promoter and GAL1 promoter.

In a preferred embodiment of the present invention, the DNAs comprise a structure in which a sense (or antisense) coding DNA that encodes a sense (or antisense) RNA of any regions of an above-described target gene mRNA, and its complementary sequence are linked in opposite directions via a spacer region, and functionally linked to a tRNA promoter.

In prokaryotes, tRNA promoters are located upstream of a gene whereas in eukaryotes, DNA regions corresponding to D loops and T loops of tRNA function as tRNA promoters. Thus, the term "tRNA promoter" of the present invention typically refers to a DNA region corresponding to the D loop or T loop of a tRNA. In general, there is more than one tRNA for every amino acid. A tRNA promoter corresponding to valine (Val) (tRNA^{Val}) can be used preferably as a tRNA promoter of the present invention.

Specifically, tRNA promoters that can be used in the present invention include promoters comprising the following nucleotide sequences.

5′-ACCGUUGGUUUCCGUAGUGUAGUGGUUAUCACGUUCGCCUAACACGCGAAAGGUCCCCGGU
UCGAAACCGGGCACUACAAAAACCAAC-3′ (SEQ ID NO: 1)

Herein, the above-mentioned phrase "functionally linked" means that a tRNA promoter is conjugated to an above-described "DNA comprising a sense coding DNA that encodes a sense RNA of any regions of a target gene mRNA, linked to its complementary sequence in opposite direction via a spacer region", for the DNA to be transcribed by the tRNA promoter. Accordingly, the tRNA promoter may be placed upstream or downstream of the DNA, however, it is usually placed upstream. An arbitrary DNA sequence can be placed between a DNA and a tRNA promoter, as long as the DNA can be transcribed.

In the present invention, when transcribed by a tRNA promoter as described above, the tRNA itself which serves as a cytoplasmic translocation signal is also linked with the above-described stem-loop RNA molecule.

In the present invention, the above-described DNAs may also contain a terminator, as necessary. There is no limitation as to the types of terminator, as long as it is a sequence that can terminate the promoter transcription. For example, terminators include known terminators, such as consecutive sequences of four or more adenine (A) nucleotides, and sequences capable of forming a palindromic structure. The DNAs of the present invention include single-stranded and double-stranded DNAs.

An above-described DNA of the present invention is transcribed by a tRNA promoter to produce a continuous transcript (an RNA molecule). The DNAs of the present invention have a structure comprising an inverted repeat sequence with an in-between spacer region, and accordingly, the DNA transcripts of the present invention also have the structure comprising an inverted repeat sequence with an in-between spacer region. Normally, in RNA molecules with such a structure, hydrogen bonds are formed between the repeat sequences, and thus a stem-and-loop structure is formed, in which the repeat sequence is the stem and the spacer region is the loop. Herein, RNA molecules that form the stem-and-loop structure are referred to as "stem-loop RNA molecules". Stem-loop RNA molecules, obtained from transcribing the above DNAs of the present invention, are also included in this invention. Herein, the phase "exhibiting RNAi effects" also applies to metabolic products and such of the stem-loop RNA molecules of the present invention, that exhibit RNAi effects, including products of RNA degradation, for example.

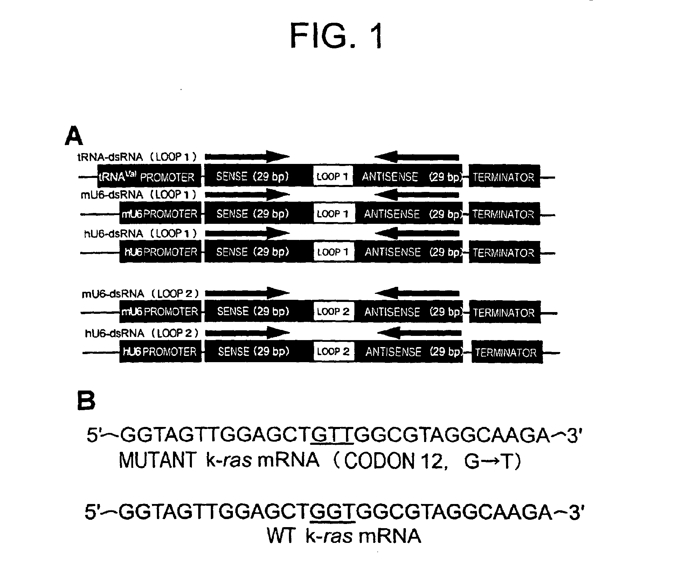

There is no limitation on the lengths of DNAs that constitute spacer regions of the present invention, as long as the repeat sequences flanking the spacer can form hydrogen bonds. Typically, the DNA length is 1-10000 nucleotides, preferably 1-100 nucleotides, more preferably 3-30 nucleotides, yet more preferably 5-20 nucleotides. There is no limitation as to the nucleotide sequences of DNAs that constitute the spacer regions, and thus an arbitrary sequence can be used as a spacer. Spacer regions are not needed when the inverted repeat sequences can form hydrogen bonds as described above. Such DNAs without spacer regions are also included in the DNAs of the present invention. Examples of the DNA structures of the present invention are schematically represented in the upper panel of Fig. 1A.

Typically, when double-stranded RNAs of about 30 bp or longer are introduced into a mammalian cell, the double-stranded RNAs are digested via the interferon (IFN) defense pathway. Thus, in the stem-loop RNA molecules of the present invention, the lengths of double-stranded RNAs of the stem region are typically 35 bp or shorter, preferably 10-35 bp, more preferably 15-30 bp, still more preferably 18-30 bp, and most preferably 26-30 bp or 31-35 bp. Likewise, in the DNAs of the present invention, the lengths of the "sense coding DNAs", or the lengths of each of the the above-described two inverted repeat sequences, are typically 35 bp or shorter, preferably 10-35 bp, more preferably 15-30 bp, still more preferably 18-30 bp, and most preferably 26-30 bp or 31-35 bp. However, there is no limitation on the lengths of the sense coding DNAs, when NMT1 promoter or GAL1 promoter is used as the promoter of the present invention, however, the lengths are preferably 10-5000 bp.

In the present invention, the double-stranded RNA portions of siRNAs (the stem portions of the stem-loop RNA molecules) may comprise perfectly paired RNAs, or unpaired portions, such as mismatches (due to uncomplimentary nucleotides), and bulges (due to missing nucleotides in either one of the pair). Such unpaired nucleotides are permissible as long as they do not hinder siRNA formation. There is no limitation on the numbers of residues in a mismatch or bulge in a stem region of the stem-loop RNA molecules expressed from the DNAs of the present invention. However, there are typically 1-10 mismatch or bulge nucleotides in a 20-bp stem region. When a promoter comprised in the DNAs of the present invention is an NMT1 promoter or GAL1 promoter, there is no limitation as to the number of nucleotides in a mismatch or bulge, and stem-loop RNA molecules expressed from the DNAs of the present invention may contain mismatchs or bulges that comprise 1-100 nucleotides in a 300-bp stem region. An example of such a mismatch can comprise 1-6 G/U pairs in a 20-bp sense strand.

Those skilled in the art can prepare the above-described DNAs of the present invention using common genetic engineering techniques. The DNAs of the present invention comprising tRNA promoters can be prepared, for example, by synthesizing desired sequences by known oligonucleotide-synthesizing methods. The double-stranded DNAs of the present invention can be obtained by synthesizing sense and antisense strands individually, and allowing them to anneal to each other.

The DNAs of the present invention can be expressed in cells by direct integration into cellular chromosomes. However, it is preferable to insert the above DNAs into a vector, which ensures efficient introduction of DNAs into cells. Vectors comprising the DNAs of the present invention are also included in this invention. The "vector" to be used herein can be selected according to the cells to be introduced with the DNAs. Vectors that may be selected for mammalian cell expression include, for example, viral vectors such as retroviral vectors, adenoviral vectors, adeno-associated viral vectors, vaccinia viral vectors, lentiviral vectors, herpes viral vectors, alpha viral vectors, EB viral vectors, papilloma viral vectors, and foamy viral vectors; and non-viral vectors such as cationic liposomes, ligand-DNA complexs, and gene guns (Niitsu, Y. et al. , Molecular Medicine, 35, 1385-1395, 1998) , but are not limited thereto.

If necessary, vectors may also contain a selection marker or such, which allows selection of cells that have been introduced with the vector. Selection markers include drug resistance markers, such as neomycin resistance gene, hygromycin resistance gene, and puromycin resistance gene; markers using enzyme activities as an indicator for selection, such as galactosidase; and markers using fluorescence emission as an indicator for selection, such as GFP. Markers that use EGF receptors or surface antigens, such as B7-2, as indicators for selection may also be used. The use of such a selection marker makes it possible to select only cells that have been introduced with the vector, specifically, cells that have been introduced with the vectors of the present invention. In addition, the a vector can prolong the duration the DNA is retained in a cell. Some vectors, for example, retroviral vectors, cause integration into chromosomes, and thus ensure a stable cellular supply of stem-loop RNA molecules from the DNAs of the present invention.

In a preferred embodiment of the present invention, the cellular expression of genes can be suppressed by introducing the DNAs of the present invention or vectors comprising the DNAs into cells. Thus, the present invention provides methods for preparing cells in which target gene expression is suppressed, comprising the steps of: introducing the DNAs of the present invention or vectors comprising the DNAs into cells, and selecting cells that have been introduced with the above vectors. In addition, the present invention provides cells that conserve the DNAs of the present invention or vectors comprising the DNAs. There is no limitation on the types of the above-described cells of the present invention, and any desired cells in which the expression of a gene is to be suppressed can be used. Even in mammalian cells where RNAi induction has been difficult, RNAi induction is now possible by using the DNAs of the present invention or vectors comprising the DNAs. The cells of the present invention, without particular limitation, include mammalian cells (for example, cells derived from mice, rats, rabbits, cows, horses, pigs, sheep, monkeys, or chimpanzees), and yeasts. Furthermore, cells in which long-term stable expression of long dsRNAs is difficult, such as plant cells, are also preferably used as cells to be introduced with the DNAs of the present invention or vectors comprising the DNAs.

Those skilled in the art can select an appropriate method, depending on the cell type, for introducing the DNAs of the present invention or vectors comprising the DNAs into the above cells. Methods for introducing DNAs into mammalian cells can be selected from, for example, calcium phosphate methods (Virology, Vol.52, p.456, 1973), electroporation methods (Nucleic Acids Res., Vol.15, p.1311, 1987), lipofection methods (J. Clin. Biochem. Nutr., Vol.7, p.175, 1989), introduction methods via viral infection (Sci. Am. , p. 34, March, 1994), gene gun, and such. Methods for introducing DNAs into plant cells can be selected from electroporation methods (Nature, Vol.319, p.791, 1986), polyethylene glycol methods (EMBO J., Vol.3, p.2717, 1984), particle gun methods (Proc. Natl. Acad. Sci. USA, Vol.85, p. 8502, 1988) , the Agrobacterium-mediated transfer methods (Nucleic. Acids Res., Vol.12, p.8711, 1984), and such.

Cells that have been introduced with the DNAs of the present invention, or vectors comprising the DNAs, can be selected by known methods, such as hybridization and PCR, using a probe or primer comprising DNA sequences specific to the DNAs of the present invention, or vectors comprising the DNAs. When the vectors that comprise the DNAs of the present invention are equipped with selection markers, such cells can also be selected by selection markers that use phenotypes as indicators.

The above cells which have been introduced with the DNAs of the present invention or vectors comprising the DNAs, are knockdown cells in which the expression of a target gene has been suppressed. Herein, the term "knockdown cell", refers to not only cells in which the expression of a target gene has been completely suppressed, but also cells in which the expression of a target gene has not been completely suppressed but only reduced. Conventionally, such cells have been created by deleting or modifying a target gene, or a regulatory region thereof. However, using the present invention makes it possible to create cells with suppressed target gene expression, by a simple method of introducing the DNAs of the present invention or vectors comprising the DNAs into cells, and selecting cells that have been introduced with the DNAs, without modifying the target gene in the chromosome. Knockdown cells thus created may be used as research materials for analyzing target gene function, and also as cell models of disease for cells that have suppressed target gene expression of a causative gene. In addition, target gene knockdown animals, animal models of disease, or such can be created by introducing the DNAs of the present invention, or vectors comprising the DNAs into germ cells, and generating individual organisms from germ cells that have retained this system. The present invention also includes the above-described knockdown cells produced by this invention, and individual organisms carrying the above-described DNAs or vectors of this invention (for example, non-human target gene knockout animals). The above-described organisms of the present invention include, for example, mice, rats, rabbits, cows, horses, pigs, sheep, monkeys, or chimpanzees.

The present invention also relates to compositions comprising DNAs of the present invention or vectors comprising the DNAs. The expression of a target gene of interest can be suppressed by the present invention, and thus a disease can be treated or prevented by suppressing the expression of a disease causative gene according to the present invention. In addition, the above-described compositions are also useful as reagents for examining functions of a gene of interest. The compositions may contain an appropriate excipient or such, when the DNAs of the present invention or vectors comprising the DNAs are used as pharmaceuticals.

In another embodiment, the present invention relates to vectors that express random stem-loop RNA molecules. The vectors comprise DNAs that have a structure, where a DNA encoding an RNA comprising random sequences, and a sequence complementary to the DNA are linked in opposite directions via a spacer region, and functionally linked to a tRNA promoter. Herein, a transcript comprising random sequences expressed from the DNAs, is referred to as a "random stem-loop RNA molecule". Search of functional genes can be achieved by introducing random stem-loop RNA molecules into cells. That is, while the DNAs of the present invention or vectors comprising the DNAs as described above can suppress the expression of a certain target gene; in the above embodiment, search for novel functional genes can be achieved by expressing the random stem-loop RNAs to suppress any gene of interest, for example, a gene whose function and sequence remain to be identified. The preferred length of the stem region of a random stem-loop RNA molecule described above is typically 30 bp or shorter, preferably 15-30 bp, more preferably 18-30 bp.

The present invention provides methods for searching functional genes, which comprise the steps of: introducing an expression vector carrying the above-described random stem-loop RNA molecule into cells; selecting cells that have been introduced with the above vector; and analyzing the phenotypes of selected cells.

In the present invention, a library may be constructed, which comprises a collection of vectors that can express random stem-loop RNA molecules comprising various random sequences. Searches for functional genes can be performed more efficiently by using the library.

In the above method, expression vectors carrying the random stem-loop RNA molecules can be introduced as described above.

In the following step of the above-described method, cells that have been introduced with the expression vector, carrying a random stem-loop RNA molecule or library, are selected, and the phenotypes of those cells are then analyzed. The phenotypic analysis can be carried out, for example, by comparing the phenotype with that of control cells, carrying no random stem-loop RNA molecule expression vectors. Such phenotypes include not only phenotypes that appear on cell surface, but also intracellular changes, for example.

If phenotypic changes of a cell are detected by the above analysis, there is a high possibility that the cell carries a random stem-loop RNA molecule that can suppress the expression of a functional gene. Therefore, to screen for functional genes, for example, probes and/or primers are constructed based on the DNA sequences, which encode the random stem-loop RNAs in the random stem-loop RNA molecule expression vectors that are carried by the cell. Functional genes can be cloned by carrying out hybridization or PCR using these probes and/or primers. Alternatively, functional genes can also be selected by database search, based on the DNA sequences that encode the random stem-loop RNAs.

The present invention also relates to siRNA expression systems, which comprise DNAs with a structure that ensures the expression of RNA molecules that exhibit RNAi effects in cells, and DNAs with a structure that ensures the expression of a polypeptide having Dicer activity (for example, Dicer protein). In a preferred embodiment of the present invention, the siRNA expression system comprises the DNAs described below in (a) and (b). The cytotoxicity of long dsRNAs can be reduced by Dicer activity.
(a) DNAs that encode stem-loop RNA molecules exhibiting RNAi effects in cells, and have a structure, in which a sense coding DNA that encodes a sense RNA of any regions of a target gene mRNA, and a sequence complementary to the DNA are linked in opposite directions via a spacer region, and functionally linked to a promoter;
(b) DNAs that comprise a structure, in which a promoter is functionally linked to a DNA that encodes a polypeptide region having the Dicer activity of a Dicer protein.

Herein, the phrase a "polypeptide that has the Dicer activity of a Dicer protein" refers to a partial polypeptide fragment that has at least the Dicer activity of a Dicer protein, or a protein comprising such polypeptides (for example, a full-length Dicer protein). The term "Dicer activity" typically refers to activities of digesting a long double-stranded RNA into double-stranded RNA fragments of 21-25 nucleotides. In general, Dicer activity can be examined by measuring RNaseIII activities. The activity can be assessed or measured by a method known to those skilled in the art, for example, an *in vitro* processing assay. For example, the *in vitro* processing assay can be carried out by the procedure described below in Examples.

There is no limitation as to the origin of Dicer protein to be used in the present invention. For example, human Dicer protein can be suitably used. For example, the nucleotide sequence of a gene that encodes Dicer protein is shown in SEQ ID NO: 55, and the amino acid sequence of Dicer protein is shown in SEQ ID NO: 56.

Examples of the above-described polypeptides of the present invention that have Dicer activity, include typically a polypeptide derived from Dicer protein, which preferably comprises the amino acid sequence of amino acids 1066-1924, more preferably of amino acids 1268-1924, still more preferably of amino acids 1296-1924 (for example, a polypeptide preferably comprising the amino acid sequence of amino acids 1066-1924, more preferably of amino acids 1268-1924, still more preferably of amino acids 1296-1924 in the amino acid sequence of SEQ ID NO: 56).

siRNA expression vectors, which contain the DNAs of (a) and (b) described above, are also included in the present invention. Those skilled in the art can readily prepare the DNAs of (a) and (b) described above by using known techniques of genetic engineering.

The present invention also provides methods for suppressing target gene expression. In a preferred embodiment of the present invention, the method for suppressing the expression of a target gene comprises expressing both a stem-loop RNA molecule, and a polypeptide having Dicer activity (for example, Dicer protein) in cells, using the siRNA expression systems or the expression vectors of the present invention. Recombinant proteins can be suitably used as Dicer in the present methods.

In another preferred embodiment of the present invention, the method for suppressing a target gene comprises a first step of treating a dsRNA, in which a sense RNA of any region of a target gene mRNA is paired with an antisense RNA, with a polypeptide having Dicer activity (for example, recombinant hDicer protein) , and a second step of introducing the resulting dsRNA into cells carrying the target gene.

The recombinant hDicer used in the above method can be produced, for example, by methods described below in Examples, but the production methods are not limited thereto. Those skilled in the art can produce the recombinant hDicer by a method appropriately modified from the methods described in Examples.

The "polypeptide having Dicer activity" in the above-described method can be, for example, a full-length Dicer protein or a protein comprising above-described partial polypeptide regions of Dicer protein. For example, the "polypeptides having Dicer activity" that are expressed from *E. coli* or insect cells can be suitably used. Those skilled in the art can express a desired protein in *E*. *coli* or insect cells by common genetic engineering techniques, and then recover and purify the protein.

Introduction of dsRNAs into cells in the above method can be readily carried out by a method known to those skilled in the art, for example, electroporation methods or lipofection methods.

There is no limitation as to the lengths of "dsRNA in which a sense RNA of any regions of a target gene mRNA is paired with an antisense RNA" in the above method. The length of a dsRNA, in which a sense RNA of any regions of a target gene mRNA is paired with an antisense RNA, or the length of a dsRNA that serves as the stem in the stem region of a stem-loop RNA molecule, is preferably at least 30 bp or longer. A dsRNA in which a sense RNA of any region of a target gene mRNA is paired with an antisense RNA, or a sense strand of the stem region of a stem-loop RNA molecule, where the dsRNA serves as the stem, may contain a mismatch or bulge of 1-100 nucleotides in a 300-bp dsRNA stem region.

Furthermore, non-human target gene knockdown animals (for example, mouse) , non-human animal models of disease or the like, can be created by introducing the DNAs or vectors of the present invention into germ cells, and generating individual organisms from those germ cells that carry the DNAs or vectors.

Those skilled in the art can create target gene knockdown animals using the DNAs or vectors of the present invention, by common techniques for creating knockout animals. One example is injecting an siRNA expression vector into eggs that are fertilized by mating an F1 male and female. Mice are grown from the fertilized eggs, and DNA is obtained from the peripheral blood of mouse tails. Then, positive founder animals that have the siRNA expression vector integrated in their chromosomes are identified, by genomic Southern blotting using a portion of the expression vector as a probe. Progeny mice are obtained by repeatedly backcrossing the F1 hybrid mice. Progeny mice with positive gene recombination are then identified by genomic Southern blotting and PCR analysis.

The above description concerns mainly cases where the siRNA expression systems are used in mammals, but the siRNA expression systems of the present invention may also be used in plants. Conventionally, RNAi was induced by introducing double-stranded RNAs directly into plant cells, but the RNAi effects were difficult to maintain due to loss of dsRNAs during cell passage. However, RNAi effects can be maintained by integrating the DNAs or vectors of the present invention into chromosomes of plant cells. In addition, plants with stable RNAi effects can be created by generating transgenic plants from such cells. Those skilled in the art can create plants by known methods.

### Brief Description of the Drawings

Fig. 1 shows the construction of dsRNA expression plasmids.
(A) The tRNA-dsRNA expression plasmid comprises the sequences of human TRNA^{Val} promoter and a terminator. The expression plasmids for mouse U6-dsRNA and human U6-dsRNA comprise mouse U6 promoter and human U6 promoter, respectively. The loop 1 sequence (5'-GAAAA-3') or loop 2 sequence (mir23 loop sequence (Lagos-Quintana, M. et al. , Science, 294, 853-858, 2001): 5'-CUUCCUGUCA-3'/ SEQ ID NO: 30) was inserted between the k-*ra*s sense strand sequence (29 bp) and the antisense strand sequence (29 bp) . The dsRNA and loop sequence in U6-dsRNA are the same as those in tRNA-dsRNA.
(B) mRNA sequences of mutant and normal k-*ras*, which are targets of the dsRNAs. The mutant k-*ras* gene has a point mutation (codon 12: GGT->GTT).

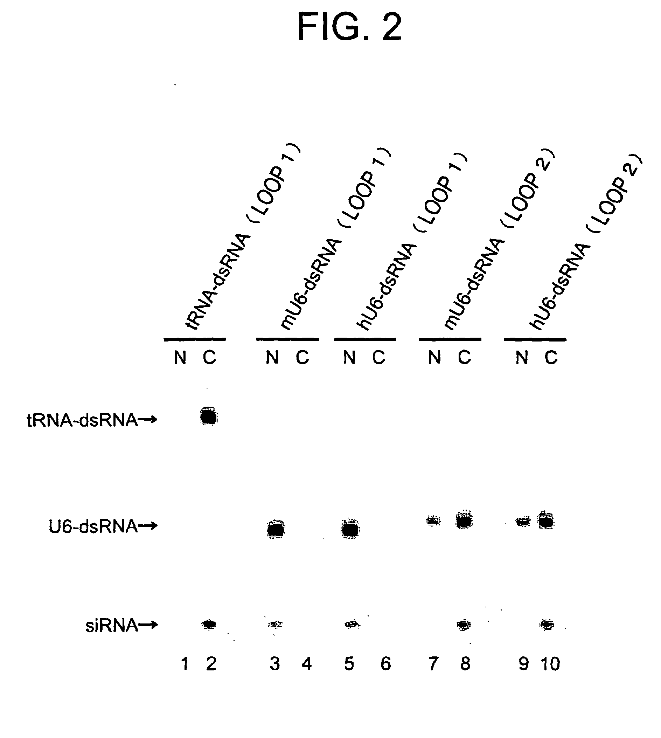

Fig. 2 shows the detection of precursor dsRNAs and siRNAs. Northern blot analysis was performed to detect precursor dsRNAs and siRNAs. A plasmid encoding tRNA-dsRNA or plasmid encoding U6-dsRNA was introduced into SW480 cells. After 48 hours, these cells were collected and separated into cytoplasmic fraction (C) and nuclear fraction (N). Total RNA was isolated from each fraction, and then fractionated on a 15% polyacrylamide gel. The Northern blotting was carried out by the procedure described herein (lane 1, nuclear fraction of cells expressing tRNA-dsRNA; lane 2, cytoplasmic fraction of cells expressing tRNA-dsRNA; lane 3, nuclear fraction of cells expressing mU6-dsRNA (loop 1) ; lane 4, cytoplasmic fraction of cells expressing mU6-dsRNA (loop 1); lane 5, nuclear fraction of cells expressing hU6-dsRNA (loop 1) ; lane 6, cytoplasmic fraction of cells expressing hU6-dsRNA (loop 1); lane 7, nuclear fraction of cells expressing mU6-dsRNA (loop 2) ; lane 8, cytoplasmic fraction of cells expressing mU6-dsRNA (loop 2); lane 9, nuclear fraction of cells expressing hU6-dsRNA (loop 2) ; lane 10, cytoplasmic fraction of cells expressing hU6-dsRNA (loop 2)).

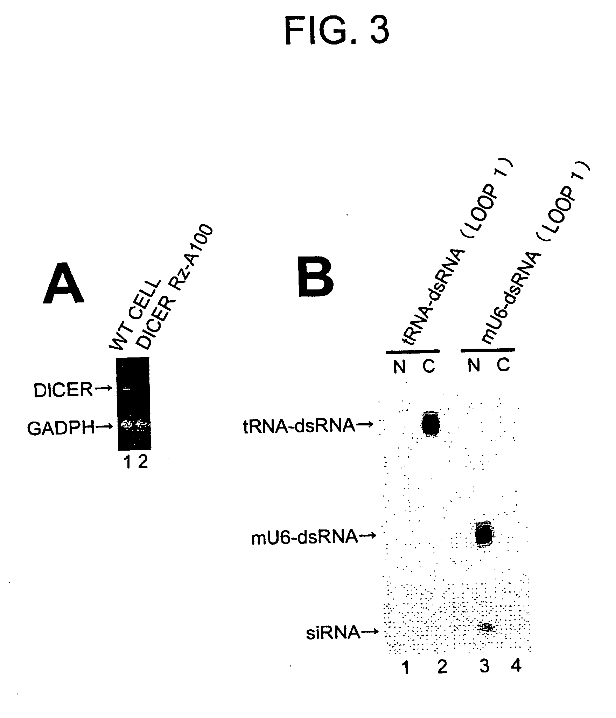

Fig. 3 shows effects of Dicer-ribozymes on dsRNA processing. (A) The expression levels of dicer genes in cells that expressed poly (A)-attached Dicer-ribozyme (Dicer-RzA100). The dicer mRNA was detected by RT-PCR with primers specific for the dicer gene (see Example 3). GADPH is an endogenous control. (B) Detection of precursor dsRNAs and siRNAs in cells that expressed Dicer-RzA100. (Lane 1, nuclear fraction of cells that expressed tRNA-dsRNA and Dicer-RzA100; lane 2, cytoplasmic fraction of cells that expressed tRNA-dsRNA and Dicer-RzA100; lane 3, nuclear fraction of cells that expressed U6-dsRNA and Dicer-RzA100; lane 4, cytoplasmic fraction of cells that expressed U6-dsRNA and Dicer-RzA100.)

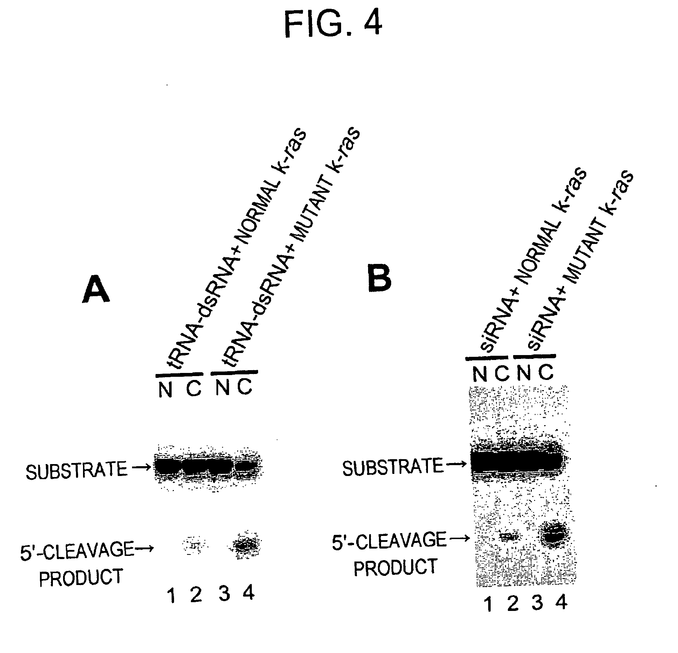

Fig. 4 shows the digestion of mutant k-*ras* mRNA by dsRNA- and siRNA-mediated degradations.
(A) Digestion of mutant k-*ra*s mRNA by cell extracts containing tRNA-dsRNA. In vitro RNAi assay is described as an experimental technique. (Lane 1, nuclear fraction of cells expressing tRNA-dsRNA and normal k-*ra*s RNA (N); lane 2, cytoplasmic fraction of cells expressing: tRNA-dsRNA and normal k-*ra*s mRNA (C); lane 3, nuclear fraction of cells expressing tRNA-dsRNA and mutant k-*ras* mRNA; lane 4, cytoplasmic fraction of cells expressing tRNA-dsRNA and mutant k-*ras* mRNA.)
(B) Digestion of mutant k-*ras* mRNA by k-*ras*-directed siRNA and SW480 cell extracts. (Lane 1, nuclear fraction of SW480 cells and normal k-*ra*s mRNA; lane 2, cytoplasmic fraction of SW480 cells and normal k-*ras* mRNA; lane 3, nuclear fraction of SW480 cells and mutant k-*ras* mRNA; lane 4, cytoplasmic fraction of SW480 cells and mutant k-*ras* mRNA.)

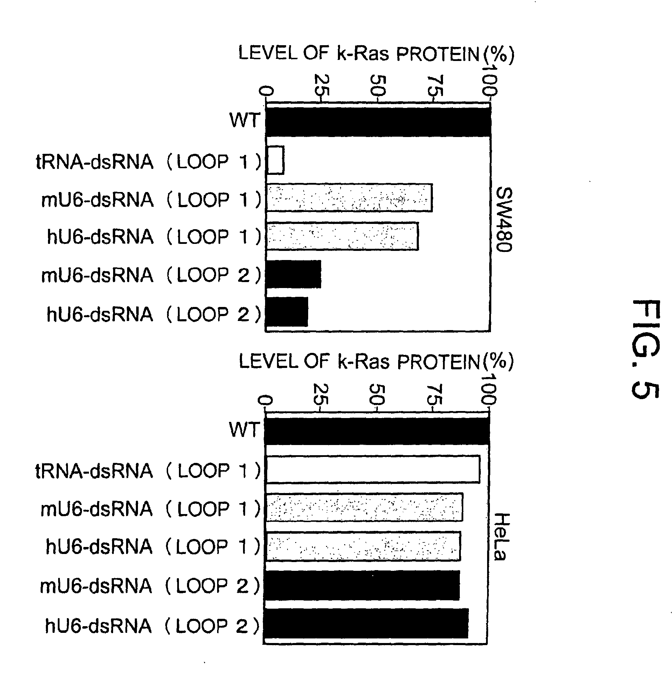

Fig. 5 shows the efficiency and specificity of RNAi by tRNA-dsRNA *in vivo.* The level of K-*ra*s protein in cells expressing tRNA-dsRNA or U6-dsRNA was analyzed by Western blotting using a k-Ras-specific antibody. The mutant k-*ras* gene was expressed in SW480 cells, whereas the normal k-*ras* gene was expressed in HeLa cells. Quantitative determination of band intensities was carried out by NIH Image Analysis using densitometry.

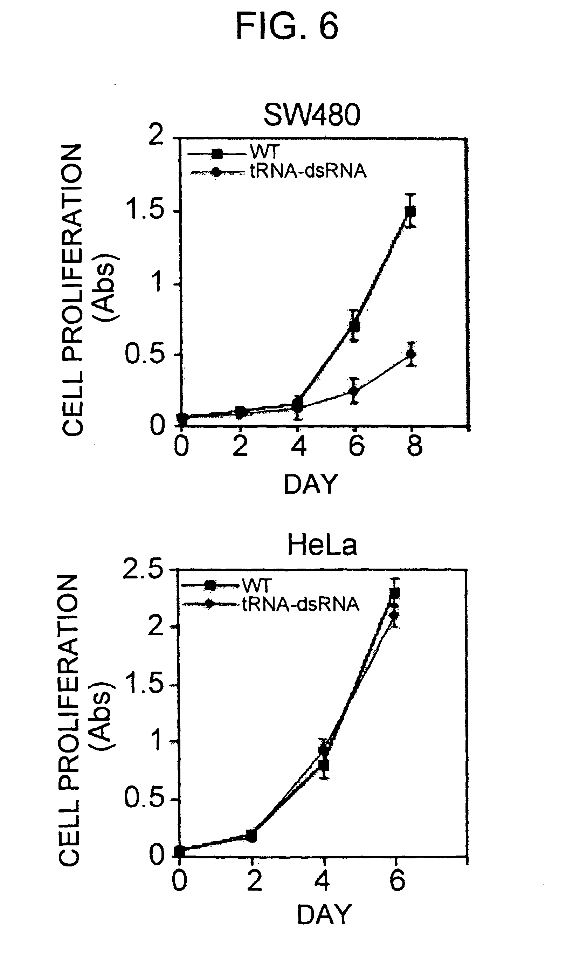

Fig. 6 shows effects of tRNA-dsRNA on cell proliferation. Proliferation rates were measured as described in Examples. Values are the means and standard deviations of three replicates in each case. Proliferation of SW480 cells that expressed tRNA-dsRNA was significantly slower than that of the wild-type SW480 cells. In contrast, the proliferation rate in HeLa cells expressing tRNA-dsRNA was the same as that of the wild-type SW480 cells.

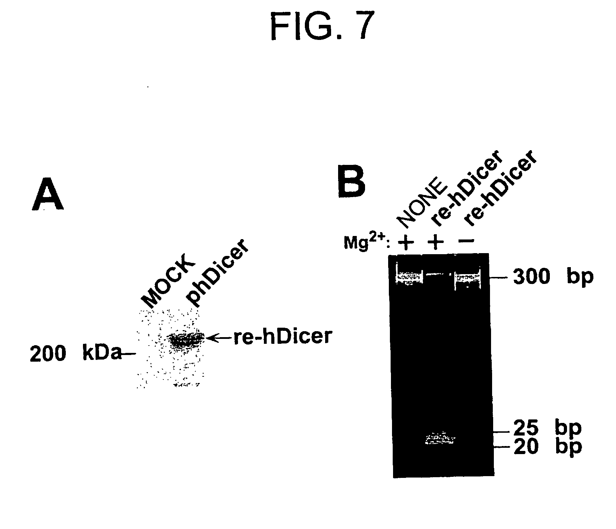

Fig. 7 (A) is a photograph showing detection of the diced siRNAs produced by hDicer. Recombinant hDicer was synthesized in *E. coli* and detected by Western blot analysis, as described in Examples. The preparation of re-hDicer (0.5 µg) was fractionated by SDS-PAGE (10% polyacrylamide) and transferred to a PVDF membrane by electroblotting. Re-hDicer was then visualized using an ECL kit and streptavidin-conjugated alkaline phosphatase. Mock: preparation of cells transfected with an empty plasmid; phDicer: preparation of cells infected with an hDicer-encoding expression plasmid. (B) is a photograph showing siRNA production by re-Dicer. Long dsRNA (10 µg) was mixed with 1 µg of re-hDicer in 200 µl of reaction buffer, with or without 5 mM MgCl₂. The reaction mixtures were incubated for 30 min at 37°C. Then 20 µl of each reaction mixture was fractionated by electrophoresis on a 12% non-denaturing polyacrylamide gel. 20-25 nt siRNAs were detected using Syber^{(R)} green II.

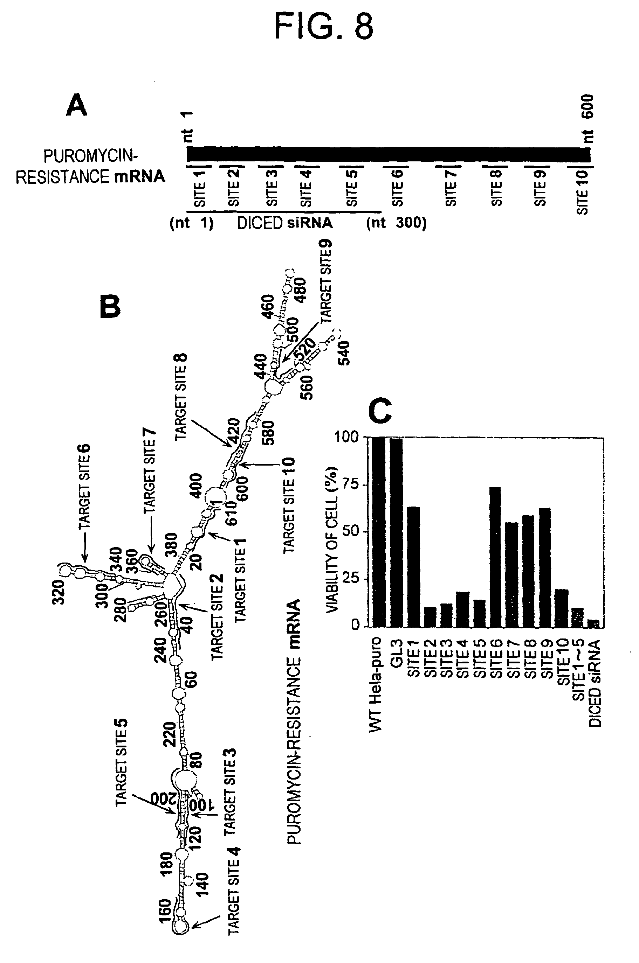

Fig. 8 shows suppression of an exogenous puromycin-resistance gene expression. (A) Ten sites (sites 1-10) were chosen as targets of synthetic siRNAs, as detailed in Examples. For preparation of diced siRNAs, long dsRNAs corresponding to the 5' region of the puromycin-resistance gene (nt. 1-300) were generated and treated with recombinant hDicer. (B) is a diagram showing the predicted secondary structure of the puromycin-resistance mRNA by the mfold program (Provost, P. et al., "Ribonuclease activity and RNA binding of recombinant human Dicer." EMBO J. 21, 5864-5874, 2002). (C) Diced siRNAs were the most effective suppressors of the exogenous puromycin-resistance gene expression (as indicated by the bar on the far right of the histogram). The transfection efficiency of siRNAs was monitored using a luciferase reporter gene from *Renilla*.

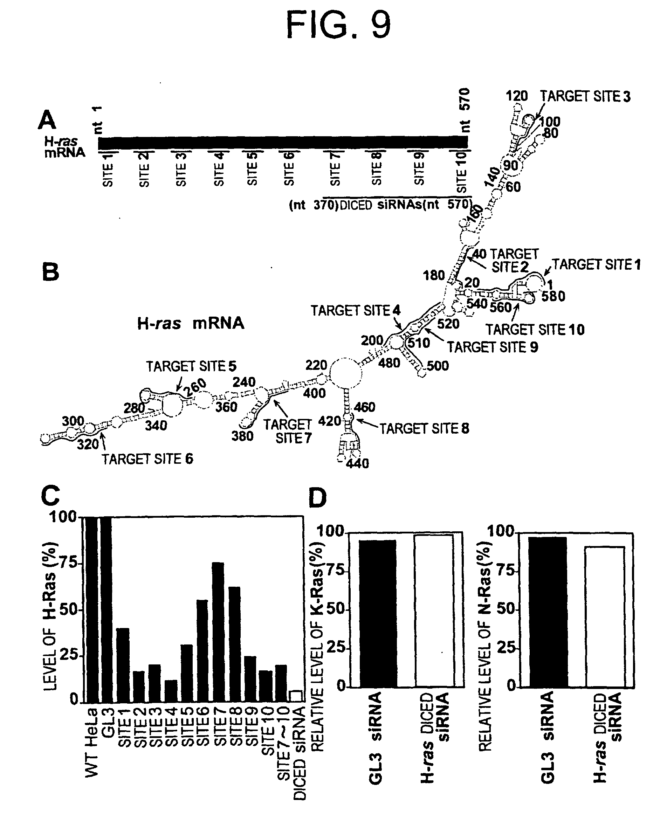

Fig. 9 shows the suppression of endogenous H-ras gene expression.
(A) As described in Examples, ten sites (sites 1-10) in H-*ras* mRNA were selected as targets of synthetic siRNAs. To prepare diced siRNAs, long dsRNA corresponding to the 3' region of the H-*ras* gene (370-570 nt), which has a low sequence homology with related ras genes, was generated and treated with recombinant hDicer. (B) is a diagram showing the deduced secondary structure of H-*ras* mRNA by mfold program.
(C) The diced siRNAs were the most effective suppressors for the expression of the endogenous H-*ras* gene (as indicated by the bar on the far right of the histogram). The results were normalized to the levels of actin control. (D) shows effects of the diced siRNAs targeting H-*ras* gene on the expression of related genes, K-*ra*s and N-*ras.* K-ras and N-ras were detected by Western blotting using specific antibodies. Expression levels were quantified by densitometry and NIH Image Analysis.

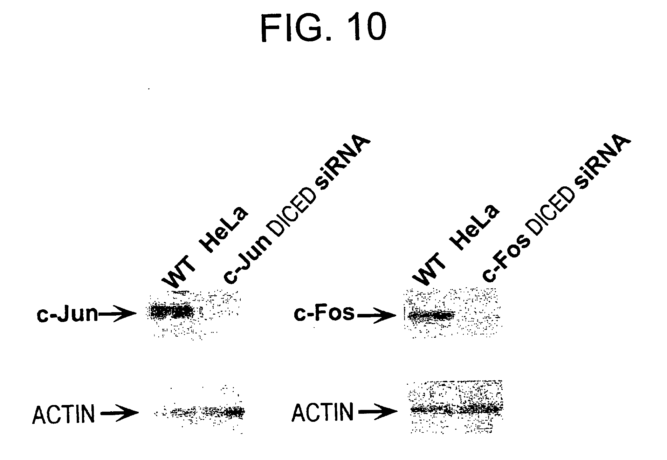

Fig. 10 shows suppression of the expression of endogenous genes c-jun and c-fos by diced siRNAs. To prepare diced siRNAs, respective long dsRNAs corresponding to the 5' regions of c-jun gene (1-200 nt) and c-fos gene (1-200 nt) were generated and treated with recombinant hDicer. Both c-Jun and c-Fos were detected by Western blotting using specific antibodies. Actin was used as an endogenous control. WT-HeLa: wild-type HeLa cell; c-Jun (c-Fos) diced siRNAs: siRNAs generated from c-jun-specific (c-fos-specific) dsRNAs.

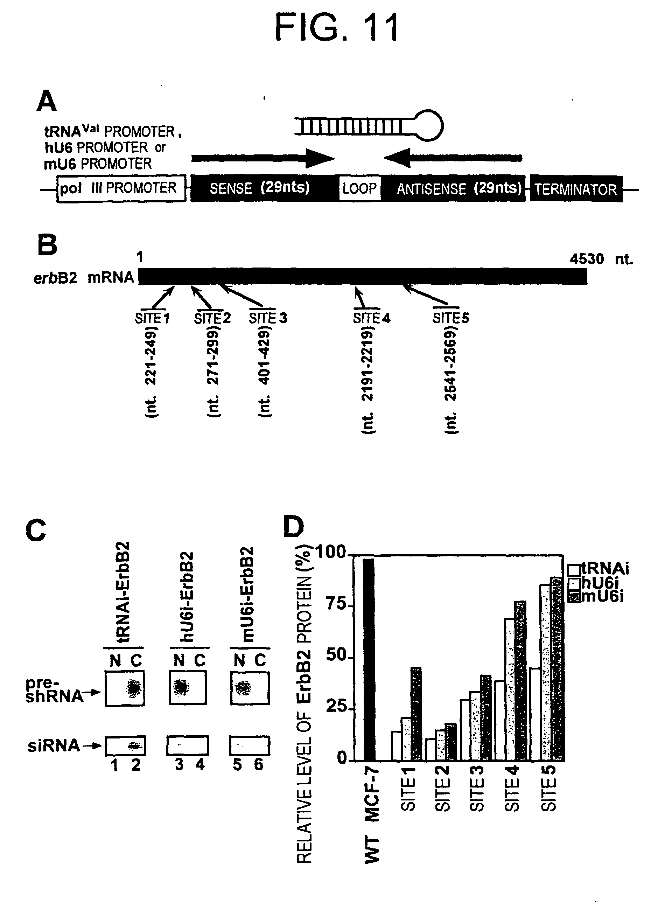

Fig. 11 shows plasmid construction for pol III-dependent expression of shRNA and its target, *erb*B2 mRNA. (A) is a diagram showing construction of shRNA expression plasmids that express shRNA under the control of a pol III promoter. The plasmids for expressing tRNA-shRNAs, hU6-shRNAs, and mU6-shRNAs, comprise a terminator sequence along with a promoter sequence from a human tRNA^{Val} gene, a human U6 promoter, and a mouse U6 promoter, respectively. A loop sequence (5'-GAAAA-3') was inserted between the sense-strand sequence (29 nts) and the antisense-strand sequence (29 nts) of the erbB2 target. (B) RNAi target sites in erbB2 mRNA. The present inventors selected five target sites of tRNA-shRNA in erbB2 mRNA. (C) is a photograph showing detection of precursor dsRNAs and siRNAs. The presence of precursor dsRNAs (pre-dsRNA) and siRNAs was analyzed by Northern blotting analysis. Plasmids encoding tRNA-shRNA, mU6-shRNA, and hU6-shRNA were introduced into MCF-7 cells. After 48 h, the cells were collected, and cytoplasmic (C) and nuclear (N) fractions were prepared. Total RNA in each fraction was isolated and fractionated on a 12% polyacrylamide gel. Northern blotting analysis was performed as described elsewhere. Lane 1, nuclear fraction of cells that expressed tRNA-shRNA; lane 2, cytoplasmic fraction of cells that expressed tRNA-shRNA; lane 3, nuclear fraction of cells that expressed hU6-shRNA; lane 4, cytoplasmic fraction of cells that expressed hU6-shRNA; lane 5, nuclear fraction of cells that expressed mU6-shRNA; lane 6, cytoplasmic fraction of cells that expressed mU6-shRNA. (D) is a graph demonstrating RNAi efficacy in MCF-7 cells by tRNA-shRNA (tRNAi) , mU6-shRNA (mU6i) , and hU6-shRNA (hU6i). The ErbB2 protein level was examined using Western blot analysis, and quantitated by densitometry. The ErbB2 protein level was normalized to the actin control.

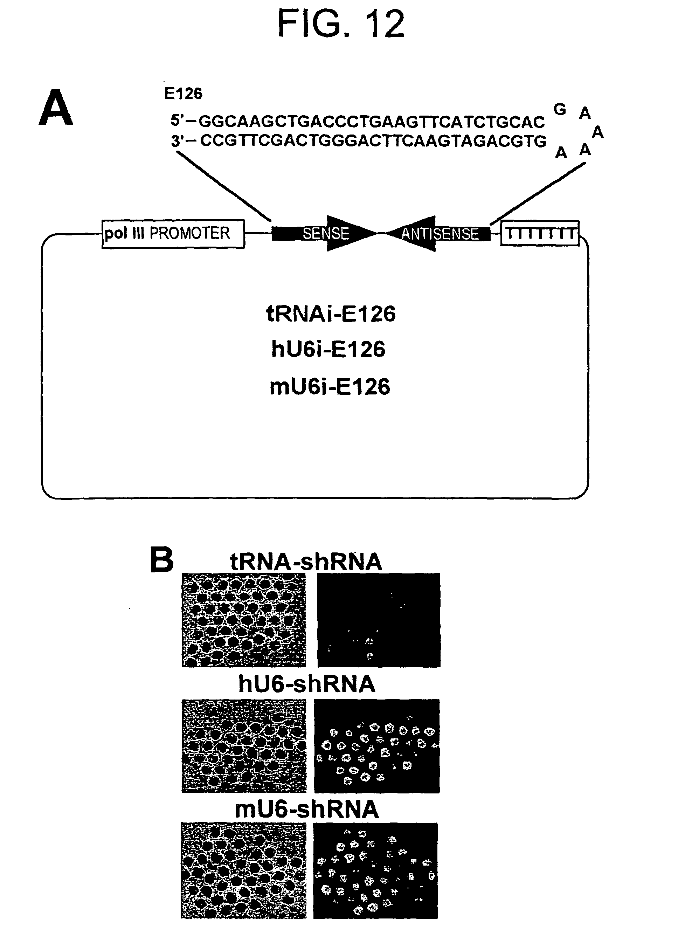

Fig. 12 demonstrates RNAi efficacy in mice by tRNA-shRNA, mU6-shRNA, and hU6-shRNA. (A) is a diagram showing construction of tRNA-shRNA, mU6-shRNA, and hU6-shRNA expression plasmids targeted against the E126 sequence in GFP mRNA. (B) shows RNAi efficacy by tRNA-shRNA, mU6-shRNA, and hU6-shRNA in eight-cell morulae. Cells were irradiated with UV light. GFP emission is detected by fluoresce microscope (Right panels). Left panels show phase-contrast photo-micrographs.

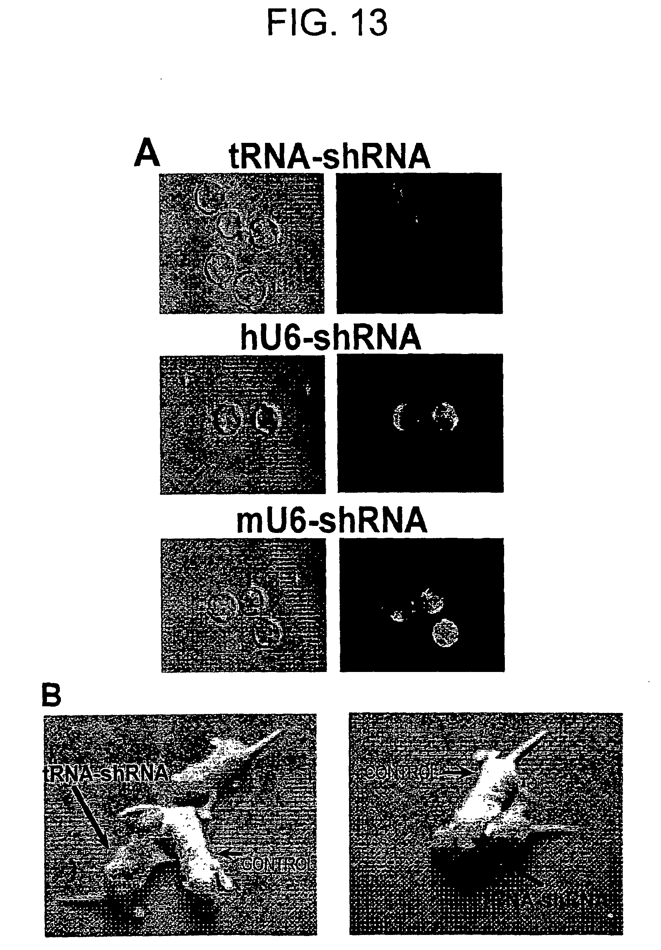

Fig. 13 shows RNAi efficacy by tRNA-shRNA, mU6-shRNA, and hU6-shRNA. (A) shows RNAi efficacy by tRNA-shRNA, mU6-shRNA, and hU6-shRNA in blastocysts. See (B) for details. (B) shows RNAi efficacy by tRNA-shRNA in newborn mice. tRNA-shRNA transgenic mice were irradiated with UV light. GFP Emission of green fluorescence was recorded with a digital card camera without filters.

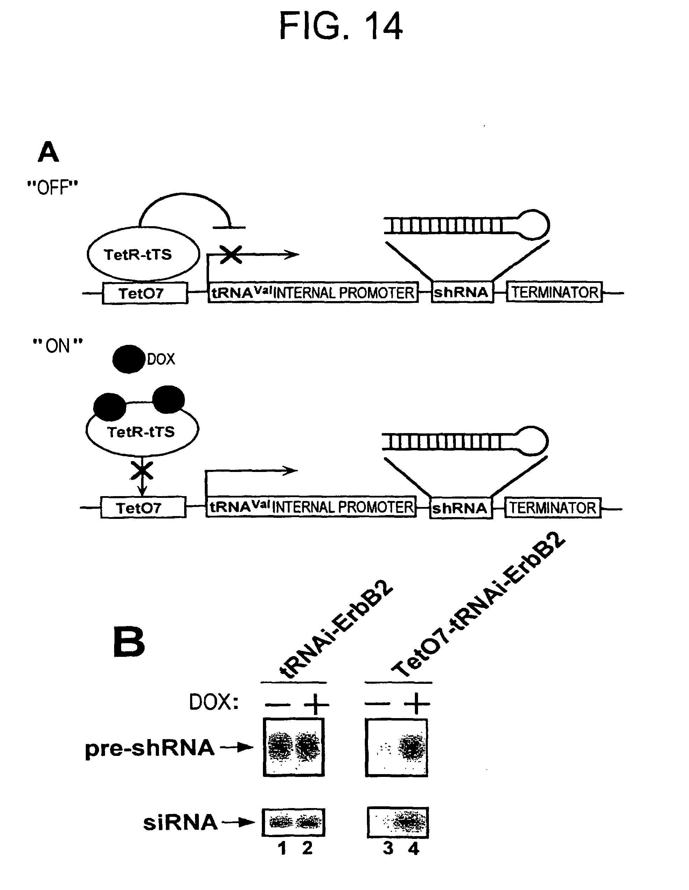

Fig. 14. shows Tetracycline-inducible (Tet-inducible) expression of tRNA-shRNA. (A) shows schematic diagrams of the Tet-dependent expression of tRNA-shRNAs. Dox, Doxycycline; Tet-tTS, tetracycline repressor; TetO7, seven copies of the tetracycline operator system. (B) shows detection of precursor dsRNAs (pre-dsRNAs) and siRNAs in the Tet-dependent shRNA expression system. The presence of precursor dsRNAs and siRNAs was analyzed by Northern blotting in the presence or absence of 100 ng/ml Dox.

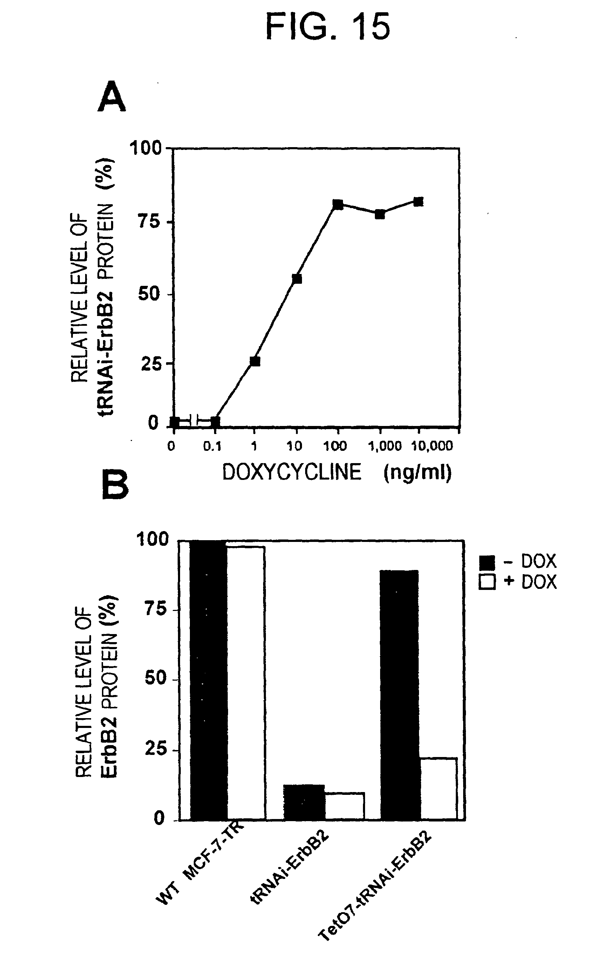

Fig. 15 shows Tetracycline-inducible (Tet-inducible) expression of tRNA-shRNA. (A) shows dose-dependency of the TetO7-tRNA-shRNA expression. There was a rapid increase in the transcript level with increasing Dox concentrations, with its maximum activation at 100 ng/ml. (B) shows effects of TetO7-tRNA-shRNA on erbB2 gene expression. The ErbB2 protein level in the presence or absence of 100 ng/ml Dox was analyzed by Western blotting and quantitated by densitometry. The ErbB2 protein level was normalized to the actin control.

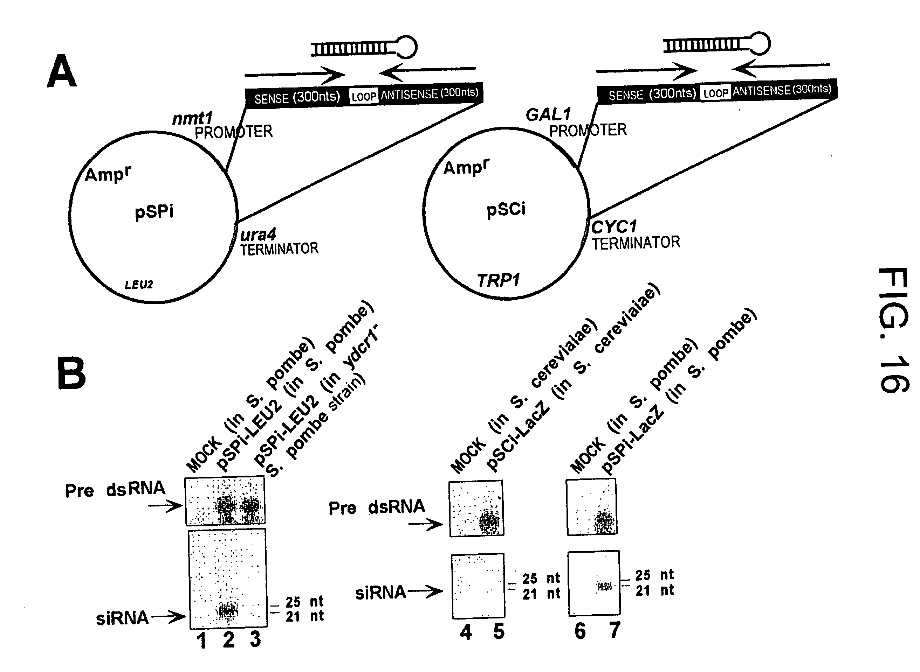

Fig. 16 (A) shows a schematic construction of dsRNA expression vectors in yeast. (B) shows dsRNA expression and siRNA production. The levels of both dsRNA and siRNA were detected by Northern blot analysis (Lane 1, Mock vector in wild-type (WT) *S. pombe* strain; lane 2, pSPi-LEU2 in WT *S. pombe* strain; lane 3, pSPi-LEU2 in *ydcr1*^{*-*} *S. pombe* strain; lane 4, Mock vector in WT *S. cerevisiae* strain; lane 5, pSCi-LacZ in WT *S. cerevisiae* strain; lane 6, Mock vector in WT *S. pombe* strain; lane 7, pSCi-LacZ in WT *S. pombe* strain). siRNAs were detected in WT *S. pombe* strain expressing pSPi-LEU2 and pSPi-LacZ. However, siRNAs were hardly detected in yDcrl⁻ *S. pombe* strain that expressed pSPi-LEU2, and in WT *S. cerevisiae* strain that expressed pSCi-LacZ.

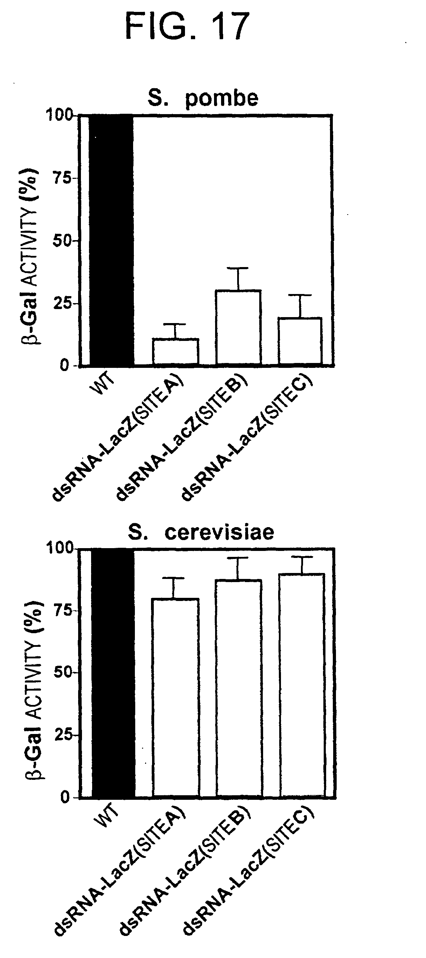

Fig. 17 shows effects of dsRNA-induced RNAi *on expression of* exogenous LacZ gene in *S. pombe* and in *S. cerevisiae.*

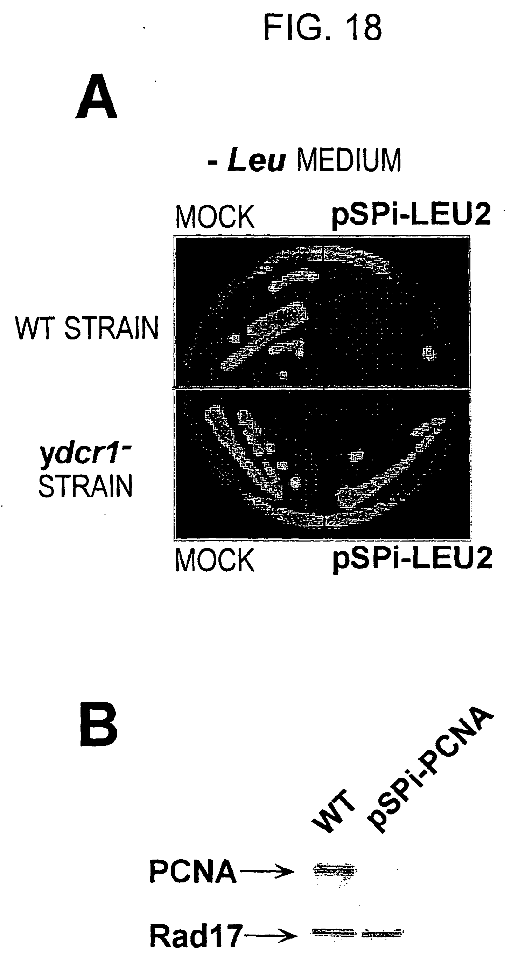

Fig. 18 shows effects of dsRNA-induced RNAi in *S. pombe.* (A) shows effects of dsRNA-induced RNAi on expression of exogenous LEU2 gene in *S. pombe*. Growth of *S. pombe* cells that expressed the dsRNA-LEU2 was examined in leucine-deficient medium (Leu⁻) . (B) shows effects of dsRNA-induced RNAi on expression of endogenous PCNA gene in *S. pombe.* The PCNA protein level was detected by Western blot analysis. Rad17 is an endogenous control.

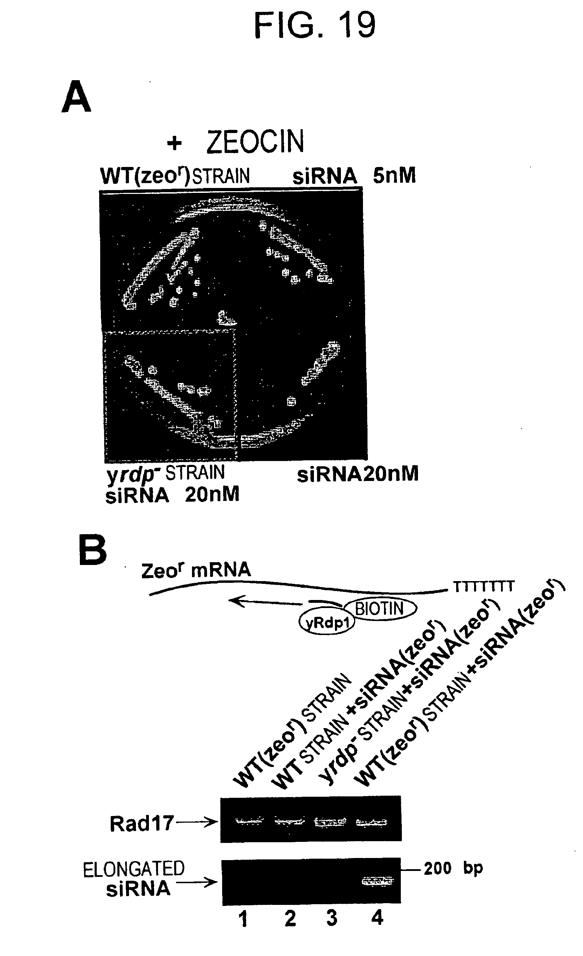

Fig. 19 shows effects of siRNA-induced RNAi in *S. pombe* and detection of RdRP activity. (A) shows effects of siRNA-induced RNAi on expression of exogenous Zeo^{r} gene in *S. pombe.* (B) shows the detection of elongated antisense siRNAs by yRdp1 complex in *S. pombe* using a biotin pull-down RT-PCR analysis (Lane 1, WT strain that expressed Zeo^{r} gene; lane 2, WT strain treated with 20 mM siRNA-Zeo^{r}; lane 3, 20 mM siRNA-Zeo^{r} in *yrdp*⁻ strain expressing Zeo^{r} gene; lane 4, Zeo^{r} expressing *S. pombe* strain treated with 20 mM siRNA-Zeo^{r}). Elongated antisense siRNAs were detected only in Zeo^{r}-expressing *S. pombe* strain that have been treated with 20 mM siRNA-Zeo^{r}. Rad17 is an endogenous control.

### Best Mode for Carrying Out the Invention

The present invention is illustrated in detail below with reference to Examples, but it is not to be construed as being limited thereto.

The materials and methods used in the experiments in Examples are described below.

### (1) Cell culture and transfection

SW480 human colon cancer cells were cultured in L-15 medium (ICN Biomedicals, Inc., Ohio, USA) supplemented with 10% FBS. HeLa cells were cultured in DMEM supplemented with 10% FBS. Transfections were performed with the Effectin^{(R)} reagent (QIAGEN, Hilden, Germany), according to the manufacturer's protocol. dsRNA-expressing SW480 and dsRNA-expressing HeLa cells were selected by puromycin incubation for two weeks.

### (2) Construction of dsRNA expression plasmids

To construct expression vectors of tRNA-dsRNA, the present inventors used a pPUR-tRNA plasmid, which comprises a chemically synthesized human gene promoter of TRNA^{Val} (Koseki, S. et al., J. Virol., 73, 1868-1877, 1999) in between the *Eco*RI and *Bam*HI sites of pPUR (Clontech, CA, USA). Chemically synthesized oligonucleotides that encode dsRNAs directed against mutant k-*ras* comprising loop 1 (5'-GAG CTC GGT AGT TGG AGC TGT TGG CGT AGG CAA GAG AAA ATC TTG CCT ACG CCA ACA GCT CCA ACT ACC GGT ACC-3'/ SEQ ID NO. 2) were amplified as double-stranded sequences by PCR. After digestion with *Sa*cI and *Kpn*I, the fragments were cloned downstream of the tRNA promoter in pPUR-tRNA. Construction of vectors to express dsRNAs from a mouse U6 promoter, except for the insertion step of dsRNA sequences, is described in the literature (Plehn-Dujowich, D., & Altman, S., Proc. Natl. Acad. Sci. USA, 95, 7327-7332, 1998; Kato, Y. et al., J. Biol. Chem., 276, 15378-15385, 2001). Chemically synthesized oligonucleotides that encode dsRNAs directed against k-*ras* comprising loop 1 (5'-GAA TTC GGT AGT TGG AGC TGT TGG CGT AGG CAA GAG AAA ATC TTG CCT ACG CCA ACA GCT CCA ACT ACC TCT AGA-3'/ SEQ ID NO. 3) or loop 2 (5'-GAA TTC GGT AGT TGG AGC TGT TGG CGT AGG CAA GAC UUC CUG UCA TCT TGC CTA CGC CAA CAG CTC CAA CTA CCC TCG AG-3'/ SEQ ID NO. 4) were amplified as double-stranded sequences by PCR, using specific up primers and down primers that comprise *Eco*RI and *Xho*I linker sequences, respectively. After digestion with *Eco*RI and *Xho*I, the fragments were cloned downstream of the mouse U6 gene promoter. In the case of a human U6 promoter, the present inventors used pTZ U6+1 (Bertrand, E. et al., RNA, 3, 75-88, 1997). Chemically synthesized oligonucleotides that encode dsRNAs directed against k-*ras* comprising loop 1 (5'-GTC GAC GGT AGT TGG AGC TGT TGG CGT AGG CAA GAG AAA ATC TTG CCT ACG CCA ACA GCT CCA ACT ACC TCT AGA-3'/ SEQ ID NO. 5) or loop 2 (5'-GTC GAC GGT AGT TGG AGC TGT TGG CGT AGG CAA GAC UUC CUG UCA TCT TGC CTA CGC CAA CAG CTC CAA CTA CCT CTA GA-3'/ SEQ ID NO. 6) were amplified as double-stranded sequences by PCR, using specific up primers and down primers that comprise *Sal*I and *Xba*I linker sequences, respectively. After digestion with *Sal*I and *Xba*I, the fragments were cloned downstream of the human U6 gene promoter, pTZ U6+1.

### (3) Construction of Dicer-directed ribozyme expression plasmids and Dicer cDNA expression plasmids

Chemically synthesized oligonucleotides that encode Dicer-directed ribozyme sequence (5'-TCC CCG GTT CGA AAC CGG GCA CTA CAA AAA CCA ACT TTC AAA GAA AGC TGA TGA GGC CGA AAG GCC GAA ACC CAT TGG GGT ACC CCG GAT ATC TTT TTT-3'/ SEQ ID NO. 7) comprising a pol III termination sequence (TTTTTT), were converted into double-stranded DNAs by PCR. After digestion with *Csp*45I and *Pst*I, the fragments were cloned downstream of the pUC-dt tRNA promoter (Koseki, S. et al., J. Virol., 73, 1868-1877, 1999; Kawasaki, H. et al. , Nature, 393, 284-289, 1998; Kawasaki, H. , & Taira, K. , EMBO rep. , 3, 443-450, 2002). To generate poly(A)-connected ribozymes, the present inventors inserted a poly(A) sequence of 100 nucleotides between the ribozyme and the pol III termination sequence (Kawasaki, H., & Taira, K., EMBO rep., 3, 443-450, 2002; Kawasaki, H. et al., Nature Biotechnol., 20, 376-380, 2002; Kawasaki, H., & Taira, K., Nucleic Acids Res., 30, 3609-3614, 2002; Warashina, M. et al., Proc. Natl. Acad. Sci. USA, 98, 5572-5577, 2001).

### (4) Preparation of the nuclear fraction and the cytoplasmic fraction of cells

SW480 cells or HeLa cells were grown to approximately 5 x 10⁶ cells and were transfected with a tRNA-dsRNA or U6-dsRNA expression vector using the Effectin^{(R)} reagent (QIAGEN, Hilden, Germany). Thirty-six hours after transfection, cells were harvested. For the preparation of the cytoplasmic fraction, collected cells were washed twice with PBS and then resuspended in digitonin lysis buffer (50 mM HEPES/KOH, pH 7.5, 50 mM potassium acetate, 8 mM MgCl₂, 2 mM EGTA, and 50 µg/mL digitonin) on ice for 10 minutes. The lysate was centrifuged at 1,000x g and the supernatant was collected as the cytoplasmic fraction. The pellets was resuspended in NP-40 lysis buffer (20 mM Tris-HCl, pH 7.5, 50 mM KC1, 10 mM NaCl, 1 mM EDTA, and 0.5% NP-40) , and kept on ice for 10 minutes. The resultant lysate was used as the nuclear fraction.

### (5) Northern blot analysis

Cytoplasmic RNAs and nuclear RNAs were extracted and purified from the cytoplasmic fraction and the nuclear fraction, respectively, using ISOGEN reagent (Wako, Osaka, Japan). Thirty micrograms of total RNA per lane were loaded on a 15% polyacrylamide gel. After electrophoresis, bands of RNA were transferred to a Hybond-N^{(R)} nylon membrane (Amersham Co., Buckinghamshire, UK). The membrane was probed with synthetic oligonucleotides that were complementary to the k-ras gene sequences. The synthetic probe was labeled with ³²P by T4 polynucleotide kinase (Takara Shuzo Co., Kyoto, Japan).

### (6) RT-PCR analysis

RT-PCR was performed using an RNA PCR Kit ver. 2 (Takara, Kyoto, Japan), plus an upstream dicer primer (nt. 1-24) and a downstream dicer primer (nt. 435-459) , or an upstream GADPH primer (nt. 230-254) and a downstream GADPH primer (nt. 442-466) as controls. The PCR products were analyzed on a 2% agarose gel by electrophoresis.

### (7) Western blot analysis

SW480 or HeLa cells that had been transfected with individual dsRNA expression vectors were harvested. Proteins were resolved by SDS-PAGE (10% polyacrylamide) and transferred to a PVDF membrane (Funakoshi Co., Tokyo, Japan) by electroblotting. Immune complexes were visualized with ECL kit (Amersham Co. , Buckinghamshire, UK) using specific polyclonal antibodies against K-Ras (UBI, CA, USA) and Actin (Santa Cruz, CA, USA).

### (8) In vitro RNAi assay

For target RNA cleavage, the present inventors synthesized a mutant k-*ras* template DNA (70 nts) and a normal k-*ras* template DNA (70 nts) using an automated DNA synthesizer. For preparation of k-*ras* mRNA substrates, the present inventors amplified template DNAs by PCR, using a k-*ras*-specific up primer that comprised a T7 promoter sequence and a k-*ras*-specific down primer. The amplified k-*ras* DNA templates were transcribed by T7 polymerase. Transcribed normal and mutant k-*ras* mRNA substrates were purified by PAGE. These mRNAs were labeled with ³²P by T4 polynucleotide kinase. For detection of the target mRNA cleavage, target mRNA (5-10 nM) was incubated with a lysate of SW480 cells that expressed either tRNA-dsRNA or U6-dsRNA under standard conditions (Zamore, P. et al. , Cell, 101, 25-33, 2000) for 2 h at 25°C. In the case where k-*ras* mRNA is the siRNA target, 100 nM siRNA was incubated with the target RNA (5-10 nM) contained in the SW480 cells lysates, at 25°C for 2 hours under standard conditions (Zamore, P. et al., Cell, 101, 25-33, 2000).

### (9) Detection of cell proliferation rates

The proliferation rate of each cell line was measured with a Cell Proliferation Kit II (Roche Ltd., Switzerland), according to the manufacturer's instructions.

### [Example 1] Construction of two types of dsRNA expression plasmids that are regulated by pol III promoters

Whether long dsRNAs are processed by Dicer in the nucleus or the cytoplasm is still unclear. Therefore, the present inventors used two types of pol III promoters (a human TRNA ^{Val} gene promoter, and a mouse U6 or human U6 promoter) to express dsRNAs in mammalian cells. If designed properly, transcripts produced using the human tRNA^{Val} gene promoter can be efficiently transported from the cytoplasm. In contrast, small RNAs transcribed under the control of a U6 promoter remained localized in the nucleus (Koseki, S. et al. , J. Virol., 73, 1868-1877, 1999). Thus, the present inventors linked TRNA^{Val} promoter (tRNA-dsRNA), mouse U6 promoter (mU6-dsRNA), or human U6 promoter (hU6-dsRNA) at its 3' end, to a DNA encoding dsRNA which serves as an extended stem-loop RNA (Fig. 1A).

The present inventors constructed dsRNA expression plasmids that target the mRNA of a mutant K-Ras containing a point mutation in codon 12 of the k-*ras* gene (Fig. 1B). The double-stranded region within the dsRNA was kept at a length of 29 bp, because long dsRNAs (>30 mers) induce non-specific reduction of mRNAs (Manche, L. et al., Mol. Cell. Biol., 12, 5238-5248, 1992; Minks, M. A. et al., J. Biol. Chem. , 254, 10180-10183, 1979). In the case of U6-based constructs, the present inventors used two types of loop motifs of stem-loop RNA. One is a loop motif that consists of 5 nucleotides (5'-GAAAA-3'), namely Loop 1 (Fig. 1A). The other is a microRNA (human *mir-23;* 48) loop motif, namely Loop 2 (Fig. 1A) . Precursor microRNAs are transported, and the processed microRNAs are thought to function in post-transcriptional gene silencing (Lagos-Quintana, M. et al., Science, 294, 853-858, 2001; Lee, Y. et al., EMBO J., 21, 4663-4670, 2002; Grishok, A. et al., Cell, 106, 23-24, 2001; Reinhart, B. J. et al., Nature, 403, 901-906, 2000; Lau, N. C. et al. , Science, 294, 858-862, 2001; Lee, R. C., & Ambros, V., Science, 294, 862-864, 2001; Mourelatos, Z. et al., Genes Dev. , 16, 720-728, 2002).

### [Example 2] Processing of tRNA^{Val} promoter-regulated dsRNAs by a Dicer complex in the cytoplasm

In RNAi, long dsRNAs are processed into short RNA duplexes of approximately 21- and 22-nt in length with staggered 3' ends in an RNase III-like reaction (Bernstein, E. et al . , Nature, 409, 363-366, 2001) . To examine whether tRNA-dsRNA and U6-dsRNA can be processed by an RNase III complex in mammalian cells, the present inventors performed Northern blotting analysis using a k-*ras* mRNA-specific probe. SW480 cells were transfected with plasmids that expressed dsRNA under the control of a tRNA^{Val} or U6 promoter. Forty-eight hours after transfection, cells were collected and separated into cytoplasmic and nuclear fractions. Total RNA in each fraction was isolated and fractionated on a 15% polyacrylamide gel. As shown in Fig. 2, in cells that expressed tRNA-dsRNA, processed siRNAs were detected in the cytoplasmic fraction, but not in the nuclear fraction. Moreover, the sequences of processed siRNAs were confirmed by cloning and sequencing of the siRNAs (data not shown).

In contrast, in cells that expressed either mU6-dsRNA or hU6-dsRNA (Loop 1) , unprocessed precursor dsRNAs were predominantly detected in the nucleus. The nuclear fraction contained very few siRNAs in cells that expressed mU6-dsRNA or hU6-dsRNA (Loop 1). However, mU6-dsRNAs (Loop 2) and hU6-dsRNAs (Loop 2) were transported into the cytoplasm and efficiently processed (Fig. 2).

A mammalian Dicer has been detected mostly in the cytoplasm by *in situ* immunostaining (Billy, E. et al. , Proc. Natl. Acad. Sci. USA, 98, 14428-14433, 2001). Therefore, tRNA-dsRNA, mU6-dsRNAs (Loop 2) and hU6-dsRNAs (Loop 2) that had been transported into the cytoplasm were thought to be processed by the Dicer-like RNase III complex.

To confirm whether tRNA-dsRNAs are processed by Dicer, the present inventors constructed a poly (A)-connected ribozyme expression plasmid directed against Dicer (Dicer-RzA100), and then introduced this plasmid into HeLa cells stably. Stable cell lines were obtained by neomycin selection. Next, to examine suppression of the dicer gene expression by Dicer-RzA100, the present inventors performed RT-PCR analysis using specific primers for the dicer mRNA. As shown in Fig. 3A, the dicer mRNA level in cells that expressed Dicer-RzA100 was reduced compared with that in WT-HeLa cells. The GADPH control showed no change in either of the cell lines. These results indicated that Dicer-RzA100 specifically cleaved dicer mRNAs.

To examine whether a reduction in Dicer affects the processing of tRNA-dsRNAs, the present inventors performed Northern blot analysis, using total RNA from cells that expressed the tRNA-dsRNAs. As shown in Fig. 3B, when the total RNA was collected from cells expressing Dicer-RzA100, siRNAs generated from tRNA-dsRNAs were not observed in either nucleus or cytoplasm. Therefore, these results suggest that tRNA-dsRNAs are processed by Dicer in the cytoplasm. In contrast, a reduction in Dicer did not affect the processing of mU6-dsRNAs (Loop 1) . Thus, mU6-dsRNAs (Loop 1) may be processed by another RNase III-like enzyme.

### [Example 3] In vitro degradation of target mRNAs using cell extracts that comprise tRNA-dsRNA, U6-dsRNA, or synthetic siRNAs

To examine the cell compartments in which target mRNA degradation by dsRNA-mediated gene silencing occurs, the present inventors performed *in vitro* RNAi assays using cell extracts that comprised tRNA-dsRNA transcripts. In these assays, the present inventors used partial mRNAs of mutant and normal k-*ras*, which had been transcribed *in vitro* by T7 polymerase, as substrates. For target mRNA cleavage, each substrate was incubated at 25°C for 2 h with an extract of SW480 cells that had been transfected with the tRNA-dsRNA expression vector. The 5'-cleavage products were resolved on sequencing gels. As shown in Fig. 4A, the mutant k-*ras* mRNA substrate was cleaved in the cytoplasmic fraction of cell extracts that comprised tRNA-dsRNA (lane 4). In contrast, in the nuclear fraction of the cell extracts, the substrate was not cleaved (lane 3). These results support an earlier report, that the RNAi-induced silencing complex (RISC) is included in a ribosomal fraction (Zamore, P. et al., Cell, 101, 25-33, 2000; Bernstein, E. et al., Nature, 409, 363-366, 2001; Hammond, S. M. et al., Science, 293, 1146-1150, 2001).

In contrast to these results, in the case of normal k-*ras* mRNA substrates, significantly smaller amounts of 5'-cleavage products were detected and these were found only in the cytoplasmic fraction of cell extracts comprising tRNA-dsRNA transcripts (Fig. 4A, lane 2). These results agree with the report, that synthesized siRNAs with one mismatched base pair in the center of the siRNA have reduced suppression efficacy of target RNAs in *in vitro* RNAi assays with lysates of *Drosophila melanogaster* (Elbashir, S. M. et al., EMBO J. , 20, 6877-6888, 2001). The present inventors confirmed that the siRNAs generated from tRNA-dsRNA, formed a mismatched base pair in the center of the siRNA, in comparison with the normal k-*ras* mRNA by sequencing analysis (data not shown). The present inventors obtained similar results with synthesized siRNAs targeting the mutant k-*ras* mRNA (Fig. 4B; Hutvagner, G., & Zamore, P. D. Science, 297, 2056-2060, 2002). Thus, the present inventors' results suggested that degradation of a target mRNA by dsRNA-mediated gene silencing also occurs in the cytoplasm, and that siRNAs are capable of recognizing one mismatched base pair in a central position.

### [Example 4] Efficiency and specificity of RNAi by tRNA-dsRNA in colon cancer cells

To examine the efficiency and specificity of RNAi by tRNA-dsRNA in human colon cancer SW480 cells, the present inventors introduced tRNA-dsRNAs and four types of U6-dsRNAs expression plasmids into SW480 and HeLa cells. The mutant k-*ras* gene was only expressed in SW480 cells (Mitchell, C. E. et al., Anal Biochem., 224, 148-153, 1995). The present inventors used HeLa cells which expressed a normal k-*ras* gene as the control. The present inventors then generated stable cell lines that expressed tRNA-dsRNAs or individual U6-dsRNAs by puromycin selection.

Next, the present inventors examined K-Ras protein levels in cells that expressed tRNA-dsRNA or individual U6-dsRNAs by Western blotting, using K-Ras-specific antibodies. For quantitation, intensities of bands were analyzed by densitometry using NIH Image Analysis. As shown in Fig. 5, the K-Ras protein level in SW480 cells that expressed tRNA-dsRNA, mU6-dsRNA (Loop 2) or hU6-dsRNA (Loop 2) was significantly lower than that in the wild-type SW480 cells. However, the K-Ras protein level in SW480 cells that expressed mU6-dsRNA (Loop 1) or hU6-dsRNA (Loop 1) was reduced only slightly compared with that in the wild-type SW480 cells. The actin was measured as the endogenous control and its level remained constant in these cell lines. Moreover, in HeLa cells that expressed tRNA-dsRNA, the K-Ras protein level was similar to that in the wild-type cells, and to that in cells that expressed the respective U6-dsRNAs. These results clearly demonstrated both the efficiency and specificity of the tRNA-dsRNA in human cancer cells.

Next, to examine the phenotype of cells that expressed tRNA-dsRNA, the present inventors analyzed the proliferation rates of various cell lines. As shown in Fig. 6, proliferation of SW480 cells expressing tRNA-dsRNA was significantly slower than that of the wild-type SW480 cells. In contrast, the proliferation rate of HeLa cells expressing tRNA-dsRNA was the same as that of the wild-type SW480 cells. These results indicated that the reduced proliferation rate of SW480 cells expressing tRNA-dsRNA correlates with the reduced protein level of K-Ras in these cells. Thus, the present inventors' tRNA-dsRNA which targets the mutant k-*ras* gene shows potential utilities as a therapeutic agent.

### [Example 5] Cloning of a human Dicer gene, and purification of recombinant human Dicer (re-hDicer)

The present inventors used human Dicer, which participates in RNAi in human cells, to prepare heterogeneous siRNAs that could target various sites in a specific target mRNA. Initially, to generate recombinant human Dicer/HERNA (hDicer) using a bacterial expression system, the present inventors used partial cDNAs of human Dicer to clone a full-length human Dicer gene.

Partial length human dicer/HERNA genes (nt. 379-1657 and nt. 1390-7037) inserted in a pBluescript vector (Stratagene, CA, USA) were a gift from Dr. S. Matsuda of University of Nagoya (Matsuda, S., Ichigotani, Y., Okuda, T., Irimura, T., Nakatsugawa, S. and Hamaguchi, M., "Molecular cloning and characterization of a novel human gene (HERNA) which encodes a putative RNA-helicase." Biochim. Biophys. Acta., 1490, 163-169, 2000). The present inventors amplified the 5' dicer-coding region (nt. 183-902) from a HeLa cDNA library by PCR with specific primers (forward primer, 5'-ATG AAA AGC CCT GCT TTG CAA CCC CT-3'/ SEQ ID NO. 8; and reverse primer, 5'-AGT TGC AGT TTC AGC ATT ACT CTT-3' / SEQ ID NO. 9) , and cloned the amplified DNA into a TA cloning vector (Invitrogen, CA, USA). The present inventors then cloned the full-length human Dicer gene from these partial cDNAs of human Dicer (hDicer). The present inventors digested the full-length hDicer cDNA, and subcloned the blunt-ended fragments at an *Eco*RV site of the PinPoint^{(R)}-Xa vector (Promega, WI, USA), which contained a biotin tag for purification of recombinant proteins. The hDicer expression plasmid was introduced into *E. coli* with 2 µM biotin, and expressed upon induction with 100 µM IPTG. Cells were then collected and pelleted by centrifugation at 5,000 rpm for 10 min. Cells were resuspended in lysis buffer [100 mM NaCl, 0.5 mM EDTA, 1% Triton X-100, 1 mM DTT, 2 mM PMSF, 50 mM Tris-HCl (pH 8.0)].

Aliquots of the re-hDicer preparation (0.5 µg) were then purified by a "pull-down" method, using streptavidin-bound SoftLink^{(R)} resin (Promega), according to the manufacturer's protocol, and was isolated from biotin tags by factor Xa protease. The hDicer was fractionated by SDS-PAGE (10% polyacrylamide) and transferred to a PVDF membrane (Funakoshi Co., Tokyo, Japan) by electroblotting. Then re-hDicer was visualized with an ECL kit (Amersham Co., Buckinghamshire, UK) and streptavidin-alkaline phosphatase.

As shown in Fig. 7A, the recombinant hDicer with a biotin tag was detected by Western blotting. The putative molecular mass of the re-hDicer was approximately 220 kDa. In contrast, a mock vector that did not encode hDicer gene did not yield the corresponding protein. Therefore, it was proven that recombinant hDicer could be produced using this expression system.

### [Example 6] In vitro processing assay

Next, to confirm whether the recombinant hDicer had RNase III activity, the present inventors performed an *in vitro* processing assay, using long dsRNAs generated by *in vitro* transcription.

To generate long dsRNA substrates, a puromycin-resistance gene (nt. 1-300), H-ras gene (nt. 370-570), c-*jun* gene (nt. 1-200) and c-*fo*s gene (nt. 1-200) were amplified by PCR using a specific forward primer comprising a T7 promoter, and a specific reverse primer comprising an SP6 promoter. Then, sense-stranded RNAs were generated by T7 RNA polymerase, and antisense-stranded RNAs were generated by SP6 RNA polymerase. All siRNAs targeting the puromycin-resistance mRNA and the H-*ras* mRNA were synthesized by Japan Bio Service (JBioS) Co. Ltd. (Saitama, Japan). These RNAs were then annealed using standard methods (Elbashir, S. M., Lendeckel, W., & Tuschl, T., "RNA interference is mediated by 21- and 22-nucleotide RNAs." Genes Dev. , 15, 188-200, 2001).

To examine hDicer activity, the present inventors mixed 10 µg of dsRNA with 1 µg of re-hDicer in a 200 µl of reaction buffer [100 mM NaCl, 20 mM HEPES, 1 mM ATP, 50 mM Tris-HCl (pH 7.0), and with or without 5 mM MgCl₂]. The mixture was incubated at 37°C for 30 min. Then, 20 µl of the reaction mixture were fractionated by electrophoresis on a non-denaturing 12% polyacrylamide gel. Bands of RNA were detected using Syber ^{(R)} green II reagent (Nippon Gene, Toyama, Japan). The present inventors recovered siRNAs of 21- to 23-nt from the reaction mixture, using a QIAquick^{(R)} nucleotide-removal kit (QIAGEN, Hilden, Germany). The siRNAs were precipitated in ethanol and then dissolved in TE buffer. The concentration of diced-siRNAs was determined by monitoring absorbance at 260 nm.

As shown in Fig. 7B, siRNAs of 20- to 25-nt were generated in the presence of recombinant hDicer. In contrast, in the absence of either recombinant hDicer or Mg²⁺ ions, no such siRNAs were detected (lanes 1 and 3). Thus, recombinant hDicer exhibited a significant activity of processing dsRNAs. Recombinant hDicer also exhibited a Mg²⁺-dependent dsRNA-processing activity. Recombinant hDicer was recently reported to exhibit ribonuclease III activity in insect cells as well (Harborth, J. et al., "Identification of essential genes in cultured mammalian cells using small interfering RNAs." J. Cell Sci., 114, 4557-4565, 2001; Provost, P. et al. , "Ribonuclease activity and RNA binding of recombinant human Dicer." EMBO J., 21, 5864-5874, 2002).

### [Example 7] Effects of diced-siRNAs targeting a puromycin-resistance gene

In mammalian cells, synthetic siRNAs of 21-nucleotides suppress the expression of both reporter genes and endogenous genes to a significant extent (Elbashir, S. M., Harborth, J., Lendeckel, W., Yalcin, A., Weber, K., & Tuschl, T., "Duplexes of 21-nucleotide RNAs mediate RNA interference in mammalian cell culture." Nature, 411, 494-498, 2001; Harborth, J., Elbashir, S. M., Bechert, K., Tuschl, T., & Weber, K., "Identification of essential genes in cultured mammalian cells using small interfering RNAs." J. Cell Sci., 114, 4557-4565, 2001; Holen, T., Amarzguioui, M., Wiiger, M. T., Babaie, E., & Prydz, H., "Positional effects of short interfering RNAs targeting the human coagulation trigger Tissue Factor." Nucleic Acids Res. , 30, 1757-1766, 2002). While long dsRNAs (>30 nts) activate PKR and 2',5'-oligoadenylate synthase, these shorter siRNAs can avoid the activation of these enzymes, and at the same time, activate the RNAi pathway. Thus, these siRNAs are useful for the sequence-specific gene silencing of gene expression. However, siRNA activities are reported to be dependent of target sites (Holen, T., Amarzguioui, M., Wiiger, M. T., Babaie, E., & Prydz, H. , "Positional effects of short interfering RNAs targeting the human coagulation trigger Tissue Factor." Nucleic Acids Res., 30, 1757-1766, 2002), and predicting the best (the most effective) target sites is extremely difficult. Since the siRNA dependence of target sites was examined only in the human tissue factor (hTF) gene (Holen, T., Amarzguioui, M., Wiiger, M. T., Babaie, E., & Prydz, H., "Positional effects of short interfering RNAs targeting the human coagulation trigger Tissue Factor." Nucleic Acids Res., 30, 1757-1766, 2002), the present inventors further examined the target site-dependence of siRNAs targeting a puromycin-resistance gene, and selected ten target sites within this gene as shown below (Figs. 8A and 8B).

Site 1: 5'-ATG ACC GAG TAC AAG CCC A-3'/ SEQ ID NO. 10;

Site 2: 5′-CTC GCC ACC CGC GAC GAC G-3′/ SEQ ID NO. 11;

Site 3: 5'-CAC CGT CGA CCC GGA CCG C-3'/ SEQ ID NO. 12;

Site 4: 5'-GAA CTC TTC CTC ACG CGC G-3'/ SEQ ID NO. 13;

Site 5: 5′-GGT GTG GGT CGC GGA CGA C-3′/ SEQ ID NO. 14;

Site 6: 5′-CAG ATG GAA GGC CTC CTG G-3′/ SEQ ID NO. 15;

Site 7: 5'-GGA GCC CGC GTG GTT CCT G-3'/ SEQ ID NO. 16;

Site 8: 5′-GGG TCT GGG CAG CGC CGT C-3′/ SEQ ID NO. 17;

Site 9: 5'-CCT CCC CTT CTA CGA GCG G-3'/ SEQ ID NO. 18;

Site 10: 5'-GCC CGG TGC CTG ACG CCC G-3'/ SEQ ID NO. 19.

The siRNA targeting GL3-luciferase was used as the control. Both the sense and antisense strands of a partial mRNA (nt. 1 to 300) of a puromycin-resistance gene were transcribed *in vitro*, using T7 and SP6 RNA polymerases, and then treated with recombinant hDicer to construct diced-siRNAs. After the *in vitro* fragmentation of dsRNAs, the diced siRNAs were purified by a gel extraction method, using QIAquick^{(R)} nucleotide-removal kit. Next, the present inventors introduced each siRNA (20 nM) and the puromycin-resistance gene expression plasmid into HeLa cells using the Oligofectamine™ reagent (Invitrogen), according to the manufacturer's instructions. The HeLa cells were cultured in DMEM supplemented with 10% FBS. After 36 h, the HeLa cells were treated with puromycin and cell viability was determined using trypan blue staining. The siRNA transfection efficiency was measured using the *Renilla* luciferase gene as a reporter gene.

As shown in Fig. 8B, in the presence of siRNAs targeting GL3-luciferase, cell viability remained unchanged compared with that of the wild-type HeLa cells. The viability of cells that had been treated with site 2-, site 3-, site 4-, site 5-, and site 10- specific siRNAs was significantly lower than that of the wild-type cells. In contrast, site 1-, site 6-, site 7-, site 8-, and site 9-siRNAs exhibited a comparatively lower inhibitory activity.

These results indicated that the efficiency of siRNAs specific for the puromycin-resistance gene depends on the target sites within a target mRNA. As anticipated, diced-siRNAs that corresponded to an unlimited number of target sites within a specific mRNA were more effective than any specific siRNA, or a mixture of site 1-, site 2, site 3-, site 4-, and site 5-siRNAs.
When the GC content of target sites and the secondary structure of the target gene were analyzed (Fig. 8B; Zuker, M. et al., "Algorithms and Thermodynamics for RNA Secondary Structure Prediction: A Practical Guide." RNA Biochemistry and Biotechnology, Barciszewski, J., & Clark, B. F. C. (eds), Kluwer Academic Publishers, Hingham, MA, USA, 1999), no clear correlation between either the secondary structure or the GC content (Table 1) , and the effectiveness of target sites was observed, as predicted by the mfold program.

**[Table 1]**

| | puromycin-resistance mRNA | | H-ras mRNA | |
|---|---|---|---|---|
| | GC (%) | inhibition (%) | GC (%) | inhibition (%) |
| target site 1 | 52.6 | 37.2 | 36.0 | 63.2 |
| target site 2 | 78.9 | 88.6 | 78.9 | 81.7 |
| target site 3 | 78.9 | 86.9 | 52.6 | 78.6 |
| target site 4 | 63.1 | 79.5 | 57.8 | 87.8 |
| target site 5 | 73.6 | 84.6 | 42.0 | 70.5 |
| target site 6 | 73.6 | 24.8 | 68.4 | 45.3 |
| target site 7 | 73.6 | 45.1 | 68.4 | 23.6 |
| target site 8 | 78.9 | 40.5 | 73.6 | 36.8 |
| target site 9 | 68.4 | 36.8 | 63.1 | 74.2 |
| target site 10 | 84.0 | 78.2 | 57.8 | 82.1 |

In the case of target genes, siRNAs targeting the 20 to 80-nt regions, and siRNAs targeting regions near the 3' end of the target gene, were more effective than the others that were tested.

### [Example 8] Effects of diced-siRNAs targeted against the H-ras gene

To confirm the effects of diced-siRNAs on expression of endogenous gene, the present inventors selected the H-*ras* gene as a target. The H-*ras* gene is a member of the *ras* gene family, which also includes K-*ras* and N-*ras* (Adjei, A. A. , "Blocking oncogenic Ras signaling for cancer therapy." J. Natl. Cancer Inst., 93, 1062-1074, 2001). Again, to examine the siRNA dependence of target sites, the present inventors selected ten target sites in the H-ras gene as shown below (Figs. 9A and 9B).

Site 1: 5'-ATG ACG GAA TAT AAG CTT G-3'/ SEQ ID NO. 20;

Site 2: 5'-GTT GGC GCC GGC GGT GTG G-3'/ SEQ ID NO. 21;

Site 3: 5′-TAC GAC CCC ACT ATA GAG G-3′/ SEQ ID NO. 22;

Site 4: 5′-AGG AGG AGT ACA GCG CCA T-3′/ SEQ ID NO. 23;

Site 5: 5′-CAA CAC CAA GTC TTT TGA G-3′/ SEQ ID NO. 24;

Site 6: 5′-GGA CTC GGA TGA CGT GCC C-3′/ SEQ ID NO. 25;

Site 7: 5′-TCT CGG CAG GCT CAG GAC C-3′/ SEQ ID NO. 26;

Site 8: 5′-GAC CCG GCA GGG AGT GGA G-3′/ SEQ ID NO. 27;

Site 9: 5′-GCT GCG GAA GCT GAA CCC T-3′/ SEQ ID NO. 28;

Site 10: 5′-GTG TGT GCT CTC CTG AGG A-3′/ SEQ ID NO. 29.

For construction of diced siRNAs, the present inventors searched for a region in the H-*ras* gene that has low homology with other ras family members. A 3'-region of the H-*ras* gene (nt. 300-500) was selected as the long dsRNA substrate, and treated with recombinant hDicer. Diced siRNAs targeting the H-*ras* gene were generated by the same method as described in Example 6. Each siRNA (20 nM) was introduced into HeLa cells, using the Oligofectamine ^{(R)} reagent (Invitrogen), according to the manufacturer's instructions. After 36 h, Hela cells transfected with each siRNA were collected, and total protein was then extracted from those cells. Protein levels of H-Ras were monitored by Western blotting using H-Ras-specific antibodies. Actin was measured as an endogenous control.

Specifically, H-Ras protein was fractionated by SDS-PAGE (10% polyacrylamide gel), and then transferred onto a PVDF membrane (Funakosi, Tokyo, Japan) by electroblotting. Immunological reactions were carried out using specific polyclonal antibodies against H-Ras, N-Ras, K-Ras, c-Jun, c-Fos, and actin as an internal control (anti-H-Ras polyclonal antibody: Oncogene Research Products, California, USA; anti-N-Ras polyclonal antibody: Oncogene Research Products; anti-K-Ras polyclonal antibody: Oncogene Research Products, anti-c-Jun polyclonal antibody: Santa Cruz Biotechnology, Santa Cruz, California, USA; anti-c-Fos polyclonal antibody: Santa Cruz Biotechnology; and anti-actin polyclonal antibody: Oncogene Research Products). Then, the resulting immune complexes were visualized by ECL^{(R)} kit. Densitometry and NIH Image Analysis were used for quantification.

As shown in Fig. 9C, the levels of H-Ras in cells that had been treated with site 2-, site 3-, site 4-, site 5-, and site 9-siRNAs, were significantly lower than that in the wild-type HeLa cells. In contrast, site 1-, site 6-, site 7-, and site 8-siRNAs exhibited a lower inhibitory activity.

When the results were normalized to the actin level, the siRNA efficiency showed to be dependent of target sites in the endogenous H-*ras* gene. In both cases of the puromycin-resistance gene and the H-*ras* gene, synthetic siRNAs targeting the central regions of the genes were less effective. As for the diced siRNAs, they were significantly more effective than all the other siRNAs tested, and also more effective than a mixture of siRNAs (site 7-, site 8-, site 9-, and site 10-siRNAs) targeting the H-*ras* gene.

The levels of related proteins, K-Ras and N-Ras, in cells that had been treated with diced-siRNAs were similar to those in the wild-type HeLa cells (Fig. 9D). Thus, the diced-siRNAs did not affect either the K-*ras* gene expression or the N-*ras* gene expression.

A similar structural analysis to the one shown in Fig. 8B was performed (Fig. 9B) , and again no correlation between the GC content (Table 1) and the degree of inhibition by siRNAs was suggested. Previous research results suggested that the effects of siRNAs, oligonucleotides, and ribozymes might share some common features (Miyagishi, M. et al., "Comparison of the suppressive effects of antisense oligonucleotides and siRNAs directed against the same targets in mammalian cells." Antisense Nucleic Acid Drug Develop., in press, 2003). More experimental data on the accessibility of target mRNAs *in vitro* and *in vivo*, rather than the computer-predicted secondary structures, as shown in Fig. 9B, for example, are likely to be needed for a more precise examination of such putative relationships. In the case of H-*ra*s, again, siRNAs targeting certain regions (20- to 280-nt, and near the 3' end) in the target gene were more effective than others, and diced siRNAs were again much more effective than individual siRNAs or a mixture of such siRNAs (Fig. 9C). These results resembled those obtained with the puromycin-resistance gene. The higher activity of the diced-siRNAs compared with individual siRNAs might have simply been due to an additive effect of targeting multiple sites on a messenger RNA. However, the results obtained with a mixture of siRNAs (site 7-, site 8-, site 9-, and site 10-siRNAs) failed to support this explanation. The siRNA effects are likely to be altered, even by a slight change in the target site (a few nucleotides) , and thus, diced-siRNAs seemed to comprise some very effective siRNA populations than the combined individual siRNAs. These results suggest that diced-siRNAs are clearly more advantageous than individual synthetic siRNAs.

### [Example 9] Effects of diced siRNAs on target c-jun mRNA or c-fos mRNA

In addition, to test the effects of diced siRNAs, the present inventors constructed corresponding diced siRNAs for c-jun mRNA or c-fos mRNA. First, long dsRNAs corresponding to the 5' region of c-jun gene (1-200 nt) or c-fos gene (1-200 nt) were prepared, and then treated with recombinant hDicer. The diced siRNAs were prepared by the same method as described in Example 6. Then, the diced siRNAs were introduced into HeLa cells. After 36 hours, the cells were harvested and total proteins were extracted from each cell. The protein levels of c-Jun and c-Fos were determined by Western blotting, using antibodies specific to the respective proteins.

As shown in Fig. 10, the level of c-Jun in cells that had been transfected with diced-siRNAs was significantly lower than that in the wild-type HeLa cells. The actin levels were similar in both transfected and wild-type cells. The same results were obtained using diced-siRNAs directed against c-fos mRNA.

These results indicated that the high potential utility of diced-siRNAs in the silencing of specific genes in human cells.

### [Example 10] Construction of plasmids encoding various shRNAs

The present inventors constructed expression vectors for three types of tRNA-shRNA, namely, tRNA^{Val} promoter-based shRNA (tRNAi), human U6 promoter-based shRNA (hU6i), and mouse U6 promoter-based shRNA (mU6i). The present inventors used the pPUR-tRNA plasmid, which contains a synthetic promoter of the human tRNA^{Val} gene between *Eco*RI and *Bam*HI sites of pPUR (Clontech, CA, USA). All constructs target the transcripts of human *erb*B2 gene (Fig. 11A).

The present inventors selected five target sites in *erb*B2 mRNA (Site 1: 5'-AAG UGU GCA CCG GCA CAG ACA UGA AGC UG-3'/ SEQ ID NO. 31, Site 2: 5'-ACC CAC CUG GAC AUG CUC CGC CAC CUC UA-3' /SEQ ID NO. 32, Site 3: 5'-UGC UCA UCG CUC ACA ACC AAG UGA GGC AG-3' / SEQ ID NO. 33, Site 4: 5'-AAG AUC CGG AAG UAC ACG AUG CGG AGA CU-3' / SEQ ID NO. 34, Site 5: 5'-GAC ACA GCU UAU GCC CUA UGG CUG CCU CU-3' / SEQ ID NO. 35), and one site in GFP mRNA (5'-GGC AAG CUG ACC CUG AAG UUC ATC TGC AC-3' / SEQ ID NO. 36) . Chemically synthesized oligonucleotides encoding sense-stranded (29 nts) and antisense-stranded (29 nts) *erb*B2 gene linked to the loop sequence (5'-GAAAA-3' ) , were amplified as double-stranded sequences by PCR using specific up primers and down primers. After digestion with *Sac*I and *Kpn*I , the fragments were cloned downstream of the tRNA gene promoter in pPUR-tRNA. The vector construction for expressing dsRNAs from mU6 and hU6 promoters is described in the literature (Tuschl, T., "Expanding small RNA interference." Nature Biotechnol. 20, 446-448, 2002; Kato, Y., Kuwabara, T., Warashina, M., Toda, H., & Taira, K., "Relationships between the activities *in vitro* and *in vivo* of various kinds of ribozyme and their intracellular localization in mammalian cells." J. Biol. Chem. 276, 15378-15385, 2001; Ohkawa, J., & Taira, K., "Control of the functional activity of an antisense RNA by a tetracycline-responsive derivative of the human U6 snRNA promoter." Human Gene Therapy 11, 577-585, 2000).

### [Example 11] Cell culture and transfection

MCF-7 human breast cancer cells were cultured in MEM (ICN Biomedicals, Inc., USA) supplemented with 10% FBS, 1% Non-Essential amino acids solution and 1 mM Sodium Pyruvate.

MCF-7 cells were grown to approximately 5 x 10⁶ cells/ 10 cm dish, and then transfected with a tRNA-dsRNA, hU6-dsRNA, or mU6-dsRNA expression vector, using the Effectin™ reagent (QIAGEN).

Stable tRNA- or U6-shRNA-expressing MCF-7 cells were selected by a two-week incubation with puromycin.

### [Example 12] Preparation of the nuclear and cytoplasmic fractions of cells

Cells were harvested 36h after transfection. For the preparation of cytoplasmic fraction, collected cells were washed twice with PBS, and then resuspended in digitonin lysis buffer (50 mM HEPES/KOH (pH 7.5) , 50 mM potassium acetate, 8 mM MgCl₂, 2 mM EGTA and 50 µM/mL digitonin) on ice for 10 min. The lysate was centrifuged at 1,000x g and the supernatant was collected as the cytoplasmic fraction. The pellet was resuspended in NP-40 lysis buffer (20 mM Tris-HC1 (pH 7.5), 50 mM KCl, 10 mM NaCl, 1 mM EDTA and 0.5% NP-40) and kept on ice for 10 min. The resultant lysate was used as the nuclear fraction.

### [Example 13] Confirmation of shRNA expression in MCF-7 cells

Expression of the various shRNAs was then confirmed in the transformed MCF-7 cells obtained at Example 11. Cytoplasmic RNAs and nuclear RNAs were extracted and purified from the cytoplasmic fraction and the nuclear fraction, respectively, using ISOGEN™ reagent (Wako, Osaka, Japan). Thirty micrograms of total RNA per lane were loaded on a 12% polyacrylamide gel for electrophoresis, and bands of RNA were then transferred to a Hybond-N™ nylon membrane (Amersham Co., Buckinghamshire, UK). The membrane was probed with synthetic oligonucleotides that have sequences complementary to the *erb*B2 gene (5'- AAG TGT GCA CCG GCA CAG ACA TGA AGC TG-3' / SEQ ID NO. 37). Each synthetic probe was labeled with the AlkPhos Direct Labelling and Detection system (Amersham Co.).

As shown in Fig. 11C, the present inventors detected both precursor shRNAs and processed siRNAs in the cytoplasm of cells that expressed tRNA-shRNA (lane 2). In contrast, in cells that expressed hU6-shRNA or mU6-shRNA, both precursor shRNAs and small amounts of siRNAs were detected in the nucleus (lanes 3 and 5), but not in the cytoplasm (lanes 4 and 6).

These results indicated that tRNA-shRNAs were effectively transported into the cytoplasm, and suggested that siRNAs might have been generated from precursor shRNAs by Dicer.

### [Example 14] Construction of tetracycline-dependent inducible system for the regulation of tRNA-shRNA expression

For construction of tetracycline-dependent inducible system, seven copies of the tetracycline operator sequence (5'-TTT ACC ACT CCC TAT CAG TGA TAG AGA AAA GTG AAA GTC GAG-3'/ SEQ ID NO. 38) were inserted downstream of the tRNA promoter in pPUR-tRNA-shRNA. To construct MCF-7-TR cell line, pTet-Tts (Clontech) that encoded a tetracycline repressor gene was introduced into the MCF-7 cells, together with pcDNA3.1-neo (Invitrogen, CA, USA) which encodes a neomycin-resistance gene. The MCF-7-TR cell line was obtained by neomycin selection. For regulation of tRNA-shRNA expression, the present inventors used doxycycline (Clontech) of various concentrations as the inducer of shRNA transcription.

### [Example 15] Effects of shRNAs on erbB2 gene expression

To examine the effects of shRNAs on *erb*B2 gene expression, namely, the effects of tRNAi-ErbB2, hU6i-ErbB2, and mU6i-ErbB2, in MCF-7 cells, the present inventors performed Western blot analysis using ErbB2-specific antibodies.

MCF-7 cells that had been transfected with vectors encoding individual shRNAs were harvested. Proteins were resolved by SDS-PAGE (10% polyacrylamide) and transferred to a PVDF membrane (Funakoshi Co., Tokyo, Japan) by electroblotting. Immune complexes were visualized with an ECL detection kit (Amersham Co. ) , using anti-ErbB2 polyclonal antibody (Oncogene, CA, USA), and polyclonal antibody against actin (Oncogene) which serves as an endogenous control. For quantitation, intensities of bands were analyzed by densitometry using NIH Image Analysis program. The ErbB2 protein level was normalized to the actin level.

As shown in Fig. 11D, the ErbB2 protein levels in MCF-7 cells that expressed tRNAi-ErbB2 (directed against sites 2 and 3), hU6i-ErbB2 (site 2) , and mU6i-ErbB2 (site 2) were significantly lower than that in the wild-type MCF-7 cells. Moreover, although the efficacy depended on the choice of target site, the effect of tRNAi-ErbB2 was at least as high as, or even higher than that of hU6i-ErbB2. Levels of actin, chosen as an endogenous control, remained constant in all cell lines. These results clearly demonstrated the usefulness of cytoplasmic tRNA-shRNAs in RNAi directed against the *erb*B2 transcript.

### [Example 16] Transgenic mice expressing tRNA-shRNA

The present inventors next examined whether the tRNA-shRNAs of the present invention are functional in mice by generating transgenic mice that expressed tRNA-shRNAs.

The present inventors used transgenic mice that carried a GFP (green fluorescent protein) gene (Okabe, M., Ikawa, M., Kominami, K., Nakanishi, T., & Nishimune, Y., " 'Green mice' as a source of ubiquitous green cells." FEBS Lett., 407, 313-319, 1997), and used this gene as the target of tRNA-shRNA (tRNAi-E126), hU6-shRNA (hU6-E126), and mU6-shRNA (mU6-E126) (Fig. 2A; E126 is part of the sequence of the gene for GFP) . *Sal*I fragments of plasmids that encoded tRNAi-E126, mU6-E126, and mU6-E126, were injected separately into wild-type eggs that had been fertilized with the sperm from a GFP-expressing male mouse (Baer, M., Nilsen, T. W., Costigan, C., & Altman, S. "Structure and transcription of a human gene for H1 RNA, the RNA component of human RNase P." Nucleic Acids Res. 18, 97-103, 1990). Then, one hundred DNA-injected eggs were transplanted into pseudo-pregnant mice. Incorporation of the transgene was examined by Southern blotting analysis. Transgenic eight-cell morulae and blastocysts that expressed tRNAi-E126, mU6-E126, and mU6-E126, were allowed to develop from injected eggs, respectively. Green fluorescence was detected by UV light (254 nm) irradiation to examine the GFP expression in these cells. tRNA-shRNA-transgenic mice (three days after birth) were also irradiated with UV light (254 nm). Photographs were taken under UV light, without filters, with digital card camera (DS-505; Fujix, Japan).

As shown in Fig. 12B, when irradiated with UV light, morulae that expressed tRNAi-E126 emitted green fluorescence. In contrast, morulae that expressed mU6-E126 or mU6-E126 emitted green fluorescence. The efficacy of tRNAi-E126 was retained since similar results were obtained at the blastocyst stage (Fig. 13A). Moreover, newborn tRNAi-E126-transgenic mice emitted significantly less green fluorescence than the WT (GFP-expressing) mice (Fig. 13B). These results indicated that tRNAi-E126 had induced sequence-specific gene silencing, not only in the eight-cell morulae and blastocysts but also in newborn mice.

### [Example 17] Development of RNAi-induced expression system

In view of the potential utility of tRNAi, and with a future goal to analyze functions of housekeeping genes, the present inventors next attempted to develop a system of RNAi inducible expression. Specifically, the present inventors attempted to regulate the tRNAi expression, using TetO7 attached to the tRNA^{Val} promoter (Fig. 14 and 15). The TetO7 system functions as an "on/off" switch for gene expression.

The present inventors first constructed a vector with TetO7 attached to the tRNA^{Val} promoter, and a stable line of MCF-7 cells (MCF-7-TR) that expressed a tetracycline-responsive repressor (tTS; Clontech). Then, the present inventors examined the expression of tRNAi-ErbB2 in the presence and absence of doxycycline (Dox) , which is a related reagent of tetracycline. As shown in Fig. 14B, in the absence of Dox, the expression of tRNAi-ErbB2 was negligible in MCF-7-TR cells (lane 3). In contrast, in the presence of Dox, significant levels of both precursor shRNAs and siRNAs were detected in the cells (lane 4). These results indicate that the tetracycline-inducible system of the present invention may regulate the expression of tRNAi-ErbB2.

The present inventors also examined the dose-dependent response to Dox (Fig. 15A). Cells that expressed tRNAi-ErbB2 were cultured for 72 h with various concentrations of Dox, and then the transcript levels were analyzed by RT-PCR. Induction of tRNAi-ErbB2 was detected with as little as 1 ng/ml Dox in the culture medium. With increasing concentrations of Dox, there was a rapid increase in the transcript level, with its full activation at 100 ng/ml. This dose of Dox yielded an 80% equivalent of the activation level achieved in a system without TetO7. Moreover, at this saturating concentration of Dox, there were no apparent changes in the proliferative behavior and morphology of the cultured cells.

The present inventors confirmed the effects of TetO7-tRNAi-ErbB2 using Western blot analysis. As shown in Fig. 15B, in the absence of Dox, the ErbB2 protein level in cells carrying the TetO7-tRNAi-ErbB2 construct was similar to that in the WT MCF-7-TR cells. In contrast, in the presence of Dox, the ErbB2 protein level was significantly lower than that in the WT MCF-7-TR cells. These results indicated that the tetracycline-inducible system of the present invention might regulate the tRNAi-ErbB2 function. The effects of tRNAi-ErbB2 were, as anticipated, independent of Dox.

In the present invention, the present inventors constructed a tRNA-based RNAi expression system, in which the tRNA-shRNAs are localized in the cytoplasm.

### [Example 18] Yeast strain and culture

*S. pombe* TCP1 strain (Invitrogen, CA, USA) and *S. cerevisiae* EGY48 strain (Invitrogen, CA, USA) were used in the present invention.

Deletion strains, *ydcr1*^{*-*} and *yrdp1*^{*-*} were generated by homologous recombination of an *ura4* gene. These deletion strains were confirmed by RT-PCR analysis, using specific primers for *ydcr1, yrdp1* and *ura4* gene, respectively.

*S. pombe* that expressed the LacZ gene was prepared, using pNMT-LacZ vector which contained a LacZ gene and an *nmt* promoter.

pYes2-LacZ plasmids (Invitrogen) were introduced into the cells of *S. cerevisiae* that expressed the LacZ gene. These yeast strains were grown in Yeast extract/peptone/dextrose (YPD) medium. Synthetic dextrose (SD) media containing -Leu, -Trp or -Ura Do Supplement (Clontech, CA, USA) were used for selecting various strains that expressed dsRNAs or exogenous genes. The strains that expressed Zeocin resistance gene were selected in YPD medium containing 50 µg/ml Zeocin (Invitrogen).

### [Example 19] Construction of dsRNA expression plasmids

The present inventors constructed dsRNA expression vectors for both *S. pombe* and *S. cerevisiae* to test whether the expressed exogenous long dsRNAs induce RNAi-mediated gene silencing in *S. pombe* or *S. cerevisiae* (Fig. 16A) . The present inventors selected exogenous LEU2 gene, LacZ gene and endogenous PCNA gene as targets of the dsRNAs. The expression of LEU2-dsRNA, PCNA-dsRNA, and LacZ-dsRNA (300 nt) in *S. pombe* (pSPi) was regulated by *nmt*1 promoter, whereas the expression of LacZ-dsRNA (300 nt) in *S. cerevisiae* (pSCi) was regulated by GAL1 promoter.

To construct vectors for dsRNA expression in *S. pombe*, the present inventors used a pNMT-TOPO cloning kit (Invitrogen), which contains an *nmt* promoter. Sense DNAs encoding LEU2 (nt. 1-300) , PCNA (nt. 1050-1350) or LacZ [Site A (nt. 1-300); Site B (nt. 1000-1300); Site C (nt. 2000-2300)] were amplified using specific up primers [LEU2 (5'-ATG TCT GCC CCT ATG TCT GCC CCT-3'/ SEQ ID NO. 39); PCNA (5'-ACA ACG GTA TCT CTC TGC AGG CTA-3'/ SEQ ID NO. 40); LacZ-Site A (5'-ATG ATA GAT CCC GTC GTT TTA CAA-3'/ SEQ ID NO. 41); LacZ-Site B (5'-GAA AAT GGT CTG CTG CTG CTG AAC-3'/ SEQ ID NO. 42) ; LacZ-Site C (5'-ATG TCG CTC CAC AAG GTA AAC AGT-3'/ SEQ ID NO. 43)], and down primers carrying an XhoI site [LEU2 (5'-GAT TTT TAG TAA ACC TTG TTC AGG 3'/ SEQ ID NO. 44) ; PCNA (5'-TCG ATA TCT GGT ATA CCC AAG TGT-3'/ SEQ ID NO. 45); LacZ-Site A (5'-GGT TAC GTT GGT GTA GAT GGG CGC-3'/ SEQ ID NO. 46); LacZ-Site B (5'-CGC TCA TCG CCG GTA GCC AGC GCG-3' / SEQ ID NO. 47); LacZ-Site C (5'-CCA ATC CAC ATC TGT GAA AGA AAG-3' / SEQ ID NO. 48)]. The PCR products were cloned into pNMT-TOPO. Antisense DNAs were then amplified by PCR using the up primers carrying an XhoI site [LEU2 (5'-GAT TTT TAG TAA ACC TTG TTC AGG -3'/ SEQ ID NO. 44); PCNA (5'-TCG ATA TCT GGT ATA CCC AAG TGT-3' / SEQ ID NO. 45) ; LacZ-Site A (5'-GGT TAC GTT GGT GTA GAT GGG CGC-3' / SEQ ID NO. 46) ; LacZ-Site B (5'-CGC TCA TCG CCG GTA GCC AGC GCG-3' / SEQ ID NO. 47); LacZ-Site C (5'-CCA ATC CAC ATC TGT GAA AGA AAG-3'/ SEQ ID NO. 48)], and down primers carrying an *Sal*I site [LEU2 (5'-ATG TCT GCC CCT ATG TCT GCC CCT-3' / SEQ ID NO. 39); PCNA (5'-ACA ACG GTA TCT CTC TGC AGG CTA-3'/ SEQ ID NO. 40); LacZ-Site A (5'-ATG ATA GAT CCC GTC GTT TTA CAA-3'/ SEQ ID NO. 41); LacZ-Site B (5'-GAA AAT GGT CTG CTG CTG CTG AAC-3'/ SEQ ID NO. 42); LacZ-Site C (5'-ATG TCG CTC CAC AAG GTA AAC AGT-3'/ SEQ ID NO. 43)]. The PCR products were digested with SalI and XhoI. The fragments were inserted into the *Xho*I-*Sal*I site of pNMT-TOPO which carried the sense strand gene.

The present inventors used pYes3CT plasmids (Invitrogen) to construct LacZ [Site A (nt. 1-300); Site B (nt. 1000-1300); Site C (nt. 2000-2300) ]-dsRNA expression plasmids for *S. cerevisiae*. Sense DNAs encoding LacZ [Site A (nt. 1-300); Site B (nt. 1000-1300); Site C (nt. 2000-2300)] were amplified by PCR using specific up primers carrying a HindIII site [LacZ-Site A (5'-ATG ATA GAT CCC GTC GTT TTA CAA-3'/ SEQ ID NO. 41); LacZ-Site B (5'-GAA AAT GGT CTG CTG CTG CTG AAC-3'/ SEQ ID NO. 42); LacZ-Site C (5'-ATG TCG CTC CAC AAG GTA AAC AGT-3'/ SEQ ID NO. 43)], and down primers carrying a KpnI site [LacZ-Site A (5'-GG TTA CGT TGG TGT AGA TGG GCG C-3' / SEQ ID NO. 49) ; LacZ-Site B (5'-CG CTC ATC GCC GGT AGC CAG CGC G-3' / SEQ ID NO. 50) ; LacZ-Site C (5'-CC AAT CCA CAT CTG TGA AAG AAA G-3' / SEQ ID NO. 51)] . These amplified DNAs were then digested with *Hind*III and *Kpn*I. The fragments were cloned into *Hind*III-*Kpn*I site of the pYes3CT. Antisense DNAs encoding LacZ (Site A: 1-300 nt, Site B: 1000-1300 nt and Site C: 2000-2300 nt) were amplified by PCR using up primers carrying a NotI site [LacZ-Site A (5'-GG TTA CGT TGG TGT AGA TGG GCG C-3'/ SEQ ID NO. 46) ; LacZ-Site B (5'-CG CTC ATC GCC GGT AGC CAG CGC G-3' / SEQ ID NO. 47); LacZ-Site C (5'-CC AAT CCA CAT CTG TGA AAG AAA G-3'/ SEQ ID NO. 48)], and down primers carrying an XhoI site [LacZ-Site A (5'-ATG ATA GAT CCC GTC GTT TTA CAA-3' / SEQ ID NO. 41) ; LacZ-Site B (5'-GAA AAT GGT CTG CTG CTG CTG AAC-3'/ SEQ ID NO. 42); LacZ-Site C (5'-ATG TCG CTC CAC AAG GTA AAC AGT-3' / SEQ ID NO. 43)]. These amplified DNAs were then digested with *Not*I and *Xho*I. The fragments were cloned into the *Not*I-*Xho*I site of pYes3CT carrying the sense strand gene.

### [Example 20] dsRNA expression and siRNA production

To confirm expression of precursor dsRNAs and processing of these dsRNAs in *S. pombe* and *S. cerevisiae* cells, the present inventors performed Northern blot analysis using individual dsRNA-specific probes.

Total RNA was obtained using YeaStar RNA kit (Zymo Research, Inc., CA, USA). Thirty micrograms of total RNA per lane were loaded onto a 2.5% agarose gel. After electrophoresis, bands of RNA were transferred to a Hybond-N™ nylon membrane (Amersham Co., Buckinghamshire, UK). The membrane was probed with specific oligonucleotides that had sequences complementary to the dsRNA-LEU2 (nt. 1-300) and dsRNA-LacZ (nt. 1-300) genes. These probes were labeled by ECL labeling kit (Amersham Co., Buckinghamshire, UK).

As shown in Fig. 16B, although unprocessed precursor dsRNAs targeting LacZ mRNA were detected in *S. cerevisiae* cells that expressed the dsRNAs, siRNAs were hardly detected (Fig. 16B, lane 5). In contrast, both precursor dsRNAs and siRNAs were detected in *S. pombe* cells that expressed the dsRNAs (lane 2). Moreover, both precursor dsRNAs and siRNAs were detected in *S. pombe* cells that expressed the dsRNA-LacZ (Fig. 16B, lane 7) . In support of the results, RNase III Dicer-like protein was not found in the genomic sequence of *S. cerevisiae.* These results suggested that siRNAs are produced from the precursor dsRNAs in *S. pombe*, but not in *S. cerevisiae.*

Thus, the present inventors constructed a deletion strain of *ydcr1* to examine the role of yDcr1 in the dsRNA processing in *S. pombe* cells. A deletion strain of *ydcr1* was constructed as described in Example 18.

As the present inventors expected, siRNAs were not produced from the precursor dsRNA (Fig. 16B, lane 3) in the *ydcr1* deletion strain expressing dsRNAs. Thus, the *S. pombe* Dicer homolog, yDcr1, is required for generating siRNAs in *S. pombe* cells.

### [Example 21] Effects of dsRNA-induced RNAi on expression of exogenous LacZ gene in S. pombe and S. cerevisiae

The present inventors constructed dsRNA expression vectors targeting LacZ (pSCi-LacZ) to examine whether dsRNAs can induce sequence-specific gene silencing in *S. cerevisiae*.

The present inventors selected three dsRNA target sites in LacZ mRNA. These dsRNA expression plasmids were then introduced into *S. cerevisiae* cells that expressed LacZ gene. β-Galctosidase activity of each cell extract was measured using Yeast β-Galctosidase Reporter Assay kit (PIERCE, Rockford, IL, USA) , according to the manufacturer's protocol. Results of the three replicates from each case are shown as mean S.D..

As shown in Fig. 17, the LacZ activity level in *S. pombe* cells that expressed dsRNA-LacZ was reduced, compared with that of the wild-type *S. pombe* cells. In contrast, the LacZ activity level in *S. cerevisiae* cells expressing dsRNA-LacZ was not significantly reduced, compared with that of the wild-type *S. cerevisiae* cells. These results suggested that exogenous dsRNAs might not induce sequence-specific gene silencing in *S. cerevisiae.* Similar results were obtained with dsRNAs targeting endogenous genes in *S. cerevisiae* cells (data not shown).

### [Example 22] Effects of dsRNA-induced RNAi on expression of exogenous LEU2 gene in S. pombe

Since siRNAs were detected in *S. pombe* cells that expressed dsRNA-LEU2, the present inventors examined the growth of *S. pombe* cells in leucin-deficient medium (Leu⁻) . A dsRNA expression plasmid targeting the LEU2 mRNA (pSPi-LEU2), and a dsRNA expression plasmid targeting the LacZ mRNA (pSPi-LacZ), were introduced into *S. pombe* cells, respectively.

In Fig. 18A, wild-type (WT) cells that expressed the LEU2 gene grew normally in the Leu⁻ medium. However, cells that expressed dsRNA-LEU2 did not grow in the medium.

dsRNA-LEU2 did not induce gene silencing in the *ydcr1* deletion strain, and as a result, these cells showed growth even in the Leu⁻ medium. In addition, the LEU2 mRNA level in cells expressing dsRNA-LEU2 was significantly reduced, compared with that of the WT cells (data not shown). Thus, exogenous dsRNAs can induce RNAi-mediated gene silencing in *S. pomb*e cells, and yDcr1 is required for the dsRNA-mediated gene silencing.

### [Example 23] Effects of dsRNA-induced RNAi on expression of endogenous PCNA gene in S. pombe

To examine whether exogenous dsRNAs suppress endogenous gene expression in *S. pombe* cells, a dsRNA plasmid targeting PCNA mRNA was introduced into cells. The present inventors then examined the PCNA protein level using a mouse PCNA monoclonal antibody (Waseem, N. H., & Lane, D. P., "Monoclonal antibody analysis of the proliferating cell nuclear antigen (PCNA): Structural conservation and the detection of a nucleolar form." J. Cell Sci., 96, 121-129, 1990), and the Rad17 protein level as a endogenous control using a mouse Rad17 monoclonal antibody (Li, L., Peterson, C. A., Kanter-Smoler, G., Wei, Y. F., Ramagli, L. S., Sunnerhagen, P., Siciliano, M. J., & Legerski, R. J., "hRAD17, a structural homolog of the *Schizosaccharomyces pombe* RAD17 cell cycle checkpoint gene, stimulates p53 accumulation." Oncogene, 18, 1689-1699, 1999) by Western blot analysis.

In Western blot analysis, cell extracts were prepared from each transformant using Y-PER yeast protein extraction reagent (PIERCE), according to the manufacturer's protocol. Proteins were resolved by SDS-PAGE (10% polyacrylamide) and transferred to a PVDF membrane (Funakoshi Co., Tokyo, Japan) by electroblotting as described in the literature (Kawasaki, H., Eckner, R., Yao, T. P., Taira, K., Chiu, R., Livingston, D. M., & Yokoyama, K. K., "Distinct roles of the co-activators p300 and CBP in retinoic-acid-induced F9-cell differentiation." Nature, 393, 284-289, 1998; Kawasaki, H., Schiltz, L., Chiu, R., Itakura, K., Taira, K., Nakatani, Y., Yokoyama, K. K., "ATF-2 has intrinsic histone acetyltransferase activity which is modulated by phosphorylation" Nature, 405, 195-200, 2000). Immune complexes were visualized with ECL kit (Amersham Co.) using specific polyclonal antibodies against Mouse PCNA (ZYMED Lab. Inc., CA, USA) and Mouse Rad17 (ZYMED Lab. Inc.). These antibodies can react with PCNA and Rad17 of *S. pombe*, respectively (Waseem, N. H., & Lane, D. P., "Monoclonal antibody analysis of the proliferating cell nuclear antigen (PCNA): Structural conservation and the detection of a nucleolar form." J. Cell Sci., 96, 121-129, 1990; Li, L., Peterson, C. A., Kanter-Smoler, G., Wei, Y. F., Ramagli, L. S., Sunnerhagen, P., Siciliano, M. J., & Legerski, R. J., "hRAD17, a structural homolog of the *Schizosaccharomyces pomb*e RAD17 cell cycle checkpoint gene, stimulates p53 accumulation." Oncogene, 18, 1689-1699, 1999).

As shown in Fig. 18B, the PCNA protein level in cells expressing dsRNA-PCNA was significantly reduced, compared with that of the wild type (WT) cells. The Rad17 protein level as a control did not change in either cells. These results indicate that exogenous dsRNAs suppressed not only the expression of exogenous LEU2 gene, but also the expression of endogenous PCNA gene, by the RNAi system in *S. pombe* cells.

### [Example 24] Effects of siRNA-induced RNAi in S. pombe and RdRP activity

Next, to examine whether synthetic siRNAs can also induce sequence-specific gene silencing in *S. pombe* cells, the present inventors synthesized siRNAs targeting a zeocin™ resistance (Zeo^{r}) gene (siRNA-Zeo^{r}) .

Zeocin-directed siRNAs (Sense strand: 5'-CUG CGU GCA CUU CGU GGC CGA-3' / SEQ ID NO. 49; Antisense strand: 5'-GGC CAC GAA GUG CAC GCA GUU-3'/ SEQ ID NO. 50) were synthesized by Japan Bio Service (JBioS) Co Ltd. These RNAs were annealed using a standard method (Elbashir, S. M., Lendeckel, W., & Tuschl, T., "RNA interference is mediated by 21- and 22-nucleotide RNAs." Genes & Dev., 15, 188-200, 2001). 5-20 nM Zeocin-directed siRNAs were transfected into S. pombe cells carrying Zeocin, using Frozen-EZ Yeast Transformation II kit (Zymo Research, Inc., CA, USA), according to the manufacturer's protocol.

Next, to examine the growth of cells that had been treated with siRNA-Zeo^{r}, the present inventors selected strains that expressed zeocin-resistance gene in YPD medium containing 50 µg/ml zeocin (Invitrogen) . In Fig. 19A, WT cells that expressed the Zeo^{r} gene grew in zeocin-containing medium. However, the growth level of cells that had been treated with siRNA-Zeo^{r} (20 nM) was significantly reduced, compared with the WT cells expressing the Zeo^{r} gene.

Since the *S. pombe* homolog of RdRP (*yrdp1*) was identified in a DNA database search, the present inventors constructed *yrdp1* deletion strain to examine the role of yRdp1 in RNAi-mediated gene silencing. The *yrdp1* deletion strain was constructed as described in Example 18.

siRNA-Zeo^{r} did not induce efficient gene silencing in the *yrdp1* deletion strain (Fig. 19A; compare the growth level of the bottom right quarter and that of the bottom left quarter). Thus, these results indicate that synthetic siRNAs can also induce RNAi-mediated gene silencing in *S. pombe*, and that yRdp1 participates in siRNA-mediated gene silencing, at least partially.

### [Example 25] 5'-Biotin-labeled antisense strand siRNA "pull-down" and Reverse transcription (RT)-PCR analysis

Moreover, to confirm whether elongated antisense strand RNAs are generated by the siRNA-RdRP complex, the present inventors performed a biotin pull down-RT-PCR analysis.

At first, the present inventors synthesized 5'-biotin-conjugated siRNAs targeting the Zeo^{r} gene. 5'-Biotin-labeled Zeocin resistance gene (Zeo^{r})-siRNAs were synthesized by Japan Bio Service (JBioS) Co Ltd. These RNAs were annealed using standard methods (Fire, A., Xu, S., Montgomery, M. K. , Kostas, S. A., Driver, S. E., & Mello, C. C., "Potent and specific genetic interference by double-stranded RNA in *Caenorhabditis elegans."* Nature, 391, 806-811, 1998). 5'-Biotin-labeled Zeo^{r} -siRNAs were transfected into S. pombe cells, or into the *yrdp1* deletion strain, using Frozen-EZ Yeast Transformation II kit (Zymo Research) according to the manufacturer's protocol. After 48h, cells were collected, and total RNA from each cell was obtained using YeaStar RNA kit (Zymo Research). Biotin-labeled antisense strand siRNAs were purified, using SoftLink™ resin (Promega, USA) which contained a streptavidin resin, according to the manufacturer's protocol.

In RT-PCR analysis, elongated antisense strand RNAs were amplified using an RNA PCR Kit ver. 2 (TaKaRa, Kyoto, Japan) with Zeo^{r} upstream primer (5'-GGC CAC GAA GTG CAC GCA GTT-3'/ SEQ ID NO. 51), and downstream primer (5'-CAC CCT GGC CTG GGT GTG GGT-3'/ SEQ ID NO. 52), or Rad17 upstream primer (5'-TCT GGT TGT GGA AAG AGT ACC GCA-3' / SEQ ID NO. 53) and downstream primer (5'-CCT CTT TAC GTA GAA TAG AGC CAA G-3' /SEQ ID NO. 54) as a control. The PCR products were analyzed by electrophoresis on a 2% agarose gel.

As shown in Fig. 19B, elongated antisense strand RNAs derived from siRNAs were detected only in cells (Zeo^{r}) that contained siRNAs (lane 4). In contrast, WT cells that contained siRNA-Zeo^{r} did not contain the elongated antisense strand RNAs (lane 2). These results suggested that siRNAs were not amplified in the absence of target mRNAs.

In addition, these elongated RNAs were not produced in the *yrdp1s* deletion strain which contained siRNA-Zeo^{r} (lane 3). These results suggested that siRNAs act not only as a guide for the RNAi-induced silencing complex (RISC) , but also as a primer for the amplification of siRNAs in *S. pombe* cells.

### Industrial Applicability

The present invention provides DNAs encoding stem-loop RNAs that exhibit RNAi effects, and vectors comprising the DNAs. The use of these items makes it possible to conveniently produce knockout cells in which a gene of interest has been disrupted. In addition, the present invention is highly anticipated to be a powerful tool for studying RNAi mechanisms and other gene functions in mammalian cells, and also as a potentially useful method for treating diseases.

## Claims

1. A DNA encoding a stem-loop RNA molecule having an RNAi effect within a cell, wherein said DNA comprises (i) a cytoplasmic translocation signal sequence, and (ii) a structure in which (a) a sense coding DNA that encodes a sense RNA of any one of the regions of a target gene mRNA, and (b) a sequence complementary to said sense coding DNA are linked in opposite directions via a spacer region, and is operably linked to a promoter.

2. The DNA according to claim 1, comprising said cytoplasmic translocation signal sequence within the spacer region.

3. The DNA according to claim 1 or 2, wherein said promoter is a tRNA promoter, a pol II-type promoter, a pol III-type promoter, or a tetracycline-induced promoter.

4. The DNA according to claim 3, wherein said tetracycline-induced promoter is tetracycline-induced tRNA promoter.

5. The DNA according to claim 1 or 2, wherein said promoter is NMT1 promoter or GAL1 promoter.

6. A DNA encoding a stem-loop RNA molecule having an RNAi effect within a cell, wherein said DNA comprises a structure in which (a) a sense coding DNA that encodes a sense RNA of any one of the regions of a target gene mRNA, and (b) a sequence complementary to said sense coding DNA are linked in opposite directions via a spacer region, and is operably linked to a promoter.

7. The DNA according to claim 3, 4, or 6, wherein said tRNA promoter is TRNA^{VAL} promoter.

8. The DNA according to any one of claims 1 to 4, 6, and 7, wherein the length of the sense coding DNA is 10 to 35 bp.

9. The DNA according to any one of claims 1 to 4, 6, and 7, wherein the length of the sense coding DNA is 26 to 30 bp.

10. The DNA according to any one of claims 1 to 4, 6, and 7, wherein the length of the sense coding DNA is 31 to 35 bp.

11. The DNA according to claim 5, wherein the length of the sense coding DNA is 10 to 5000 bp.

12. The DNA according to any one of claims 1 to 11, wherein the length of the spacer region is 1 to 10000 nucleotides.

13. The DNA according to any one of claims 1 to 11, wherein the length of the spacer region is 1 to 100 nucleotides.

14. The DNA according to any one of claims 1 to 4, 6 to 10, 12, and 13, wherein the stem region of the stem-loop RNA molecule to be expressed is 10 to 35 bp in length.

15. The DNA according to any one of claims 1 to 4, 6 to 10, 12 and 13, wherein the stem region of the stem-loop RNA molecule to be expressed is 26 to 30 bp in length.

16. The DNA according to any one of claims 1 to 4, 6 to 10, 12 and 13, wherein the stem region of the stem-loop RNA molecule to be expressed is 31 to 35 bp in length.

17. The DNA according to any one of claims 1 to 16, wherein the stem-loop RNA molecule to be expressed is constructed to comprise a mismatch or bulge in the stem region.

18. The DNA according to claim 17, wherein the sense strand is constructed to comprise a mismatch having one to six G-U nucleotides for every 20 base pairs.

19. The DNA according to any one of claims 1 to 18, wherein the stem-loop RNA molecule to be expressed is constructed to comprise a mismatch or bulge of one to ten nucleotides for every 20 base pairs in the stem region.

20. The DNA according to claim 19, wherein said mismatch has one to six G-U nucleotides for every 20 base pairs in the sense strand.

21. The DNA according to claim 5 or 11, wherein the stem-loop RNA molecule to be expressed is constructed to comprise a mismatch or bulge of 1 to 100 nucleotides for every 300 base pairs in the stem region.

22. The DNA according to any one of claims 1 to 21, wherein said cell is a mammalian cell or yeast cell.

23. A stem-loop RNA molecule having an RNAi effect within a cell, wherein said RNA is a transcription product of a DNA according to any one of claims 1 to 22.

24. A vector that comprises a DNA according to any one of claims 1 to 22.

25. A cell that carries a DNA according to any one of claims 1 to 22, or the vector according to claim 24.

26. The cell according to claim 25, wherein said cell is a mammalian cell or yeast cell.

27. A composition comprising a DNA according to any one of claims 1 to 22, or the vector according to claim 24.

28. A method for producing cells in which the expression of a target gene is suppressed, wherein said method comprises the steps of introducing a DNA according to any one of claims 1 to 22, or the vector according to claim 24 into cells, and selecting cells carrying said DNA or vector.

29. A vector comprising a DNA encoding a stem-loop random RNA molecule within a cell, wherein said DNA comprises (i) a cytoplasmic translocation signal sequence, and (ii) a structure in which (a) a sense coding DNA that encodes a sense RNA of any one of the regions of a target gene mRNA, and (b) a sequence complementary to said sense coding DNA are linked in opposite directions via a spacer region, and is operably linked to a tRNA promoter.

30. An individual organism carrying a DNA according to any one of claims 1 to 22, or the vector according to claim 24 or 29.

31. The individual organism according to claim 30, which is a non-human target gene-knockout animal.

32. The individual organism according to claim 30 or 31, wherein said organism is selected from the group consisting of a mouse, rat, rabbit, cow, horse, pig, sheep, monkey, and chimpanzee.

33. The vector according to claim 29, wherein the stem region of the stem-loop random RNA molecule to be expressed is 10 to 35 bp in length.

34. A method of screening for a functional gene, wherein said method comprises the steps of:
(a) introducing the vector according to claim 29 or 33 into cells,
(b) selecting cells carrying the vector, and
(c) analyzing the phenotype of said selected cells.

35. The method of screening for a functional gene according to claim 34, which further comprises the step of screening for the functional gene based on the random sequences of a vector sequence, within a cell that is found to have altered phenotypes by phenotypic analysis.

36. An siRNA expression system that comprises:
(a) A DNA that encodes a stem-loop RNA molecule having an RNAi effect within a cell, wherein said DNA comprises a structure in which (i) a sense coding DNA that encodes a sense RNA of any one of the regions of a target gene mRNA, and (ii) a sequence complementary to said sense coding DNA are linked in opposite directions via a spacer region, and is operably linked to a promoter, and
(b) a DNA comprising a structure, in which a DNA encoding a polypeptide region having the Dicer activity of a Dicer protein, and is operably linked to a promoter.

37. The siRNA expression system according to claim 36, wherein said promoter according to (a) is a tRNA promoter or Pol III-type promoter.

38. The siRNA expression system according to claim 36, wherein said promoter according to (a) is NMT1 promoter, GAL1 promoter, or Pol II-type promoter.

39. The siRNA expression system according to claim 38, wherein the stem-loop RNA molecule to be expressed comprises a mismatch or bulge of 1 to 100 nucleotides for every 300 base pairs in the stem region.

40. The siRNA expression system according to claim 36, wherein the promoter according to (b) is Pol II-type promoter.

41. The siRNA expression system according to any one of claims 36 to 40, wherein said cell is a mammalian cell or yeast cell.

42. The siRNA expression system according to any one of claims 36 to 41, wherein the length of the dsRNA resulting from the pairing of sense RNA with antisense RNA in any one of the regions of the target gene mRNA, or the length of the stem region in the stem-loop RNA molecule that has said dsRNA as the stem, is at least 30 base pairs or more.

43. The siRNA expression system according to any one of claims 36 to 41, which comprises a mismatch or bulge of 1 to 100 nucleotides for every 300 base pairs in the dsRNA resulting from the pairing of sense RNA with antisense RNA in any one of the regions of the target gene mRNA, or in the sense strand within the stem region of the stem-loop RNA molecule that has said dsRNA as the stem.

44. An siRNA expression vector containing the DNAs of both (a) and (b) of claim 36.

45. A method for suppressing the expression of a target gene, wherein said method comprises expressing both a stem-loop RNA molecule and a polypeptide having Dicer activity, using the siRNA expression system according to any one of claims 36 to 43, or the expression vector according to claim 44.

46. A method for suppressing the expression of a target gene wherein said method comprises the steps of:
(a) treating a dsRNA that results from the pairing of sense RNA with antisense RNA in any one of the regions of the target gene mRNA, or the stem-loop RNA molecule having said dsRNA as the stem, with a polypeptide having Dicer activity, and
(b) introducing the dsRNA produced in the above-mentioned step (a) into a cell comprising the target gene.

47. The method according to claim 46, wherein said polypeptide having Dicer activity is a polypeptide comprising the amino acid sequence of positions 1066 to 1924 of Dicer protein.

48. The method according to claim 46, wherein said polypeptide having Dicer activity is a polypeptide comprising the amino acid sequence of positions 1268 to 1924 of Dicer protein.

49. The method according to claim 46, wherein said polypeptide having Dicer activity is a polypeptide comprising the amino acid sequence of positions 1296 to 1924 of Dicer protein.

50. The method according to claim 46, wherein the polypeptide having Dicer activity is the full length Dicer protein.

51. The method according to any one of claims 45 to 50, wherein said cell is a mammalian cell or yeast cell.

52. The method according to claim 46, wherein said polypeptide having Dicer activity is a polypeptide according to any one of claims 47 to 50 expressed in E. coli.

53. The method according to claim 46, wherein said polypeptide having Dicer activity is a polypeptide according to any one of claims 44 to 47 expressed in insect cells.

54. The method according to any one of claims 45 to 53, wherein said dsRNA resulting from the pairing of sense RNA with antisense RNA in any one of the regions of the target gene mRNA, or the stem region of the stem-loop RNA molecule that has said dsRNA as the stem, has a length of at least 30 base pairs or more.

55. The method according to any one of claims 45 to 53, comprising a mismatch or bulge of 1 to 100 nucleotides for every 300 base pairs in the dsRNA that results from the pairing of sense RNA with antisense RNA in any one of the regions of the target gene mRNA, or in the sense strand within the stem region of the stem-loop RNA molecule that has said dsRNA as the stem.


SEQUENCE LISTING

<110>  NATIONAL INSTITUTE OF ADVANCED INDUSTRIAL SCIENCE AND TECHNOLOGY

<120>  EXPRESSION SYSTEM FOR STEM-LOOP RNA MOLECULES HAVING RNAi EFFECT

<130>  GFU-A0203Y1P

<150>  JP 2002-127089
<151>  2002-04-26

<150>  JP 2003-4706
<151>  2003-01-10

<150>  US 60/449,860
<151>  2003-02-27

<160>  56

<170>  PatentIn version 3.1

<210>  1
<211>  88
<212>  RNA
<213>  Homo sapiens

<400> 1

accguugguu uccguagugu agugguuauc acguucgccu aacacgcgaa agguccccgg 60

uucgaaaccg ggcacuacaa aaaccaac 88

<210> 2

<211> 75

<212> DNA

<213> Artificial

<220>

<223> an artificially synthesized sequence

<400> 2

gagctcggta gttggagctg ttggcgtagg caagagaaaa tcttgcctac gccaacagct 60

ccaactaccg gtacc 75

<210> 3

<211> 75

<212> DNA

<213> Artificial

<220>

<223>  an artificially synthesized sequence

<400>  3
gaattcggta gttggagctg ttggcgtagg caagagaaaa tcttgcctac gccaacagct      60

ccaactacct ctaga                                                        75

<210>  4
<211>  80
<212>  DNA
<213>  Artificial

<220>
<223>  an artificially synthesized sequence

<400>  4
gaattcggta gttggagctg ttggcgtagg caagacuucc ugucatcttg cctacgccaa      60

cagctccaac taccctcgag                                                   80

<210>  5
<211>  75
<212>  DNA
<213>  Artificial

<220>

<223>  an artificially synthesized sequence

<400>  5
gtcgacggta gttggagctg ttggcgtagg caagagaaaa tcttgcctac gccaacagct      60

ccaactacct ctaga                                                        75

<210>  6
<211>  80
<212>  DNA
<213>  Artificial

<220>

<223>  an artificially synthesized sequence

<400>  6
gtcgacggta gttggagctg ttggcgtagg caagacuucc ugucatcttg cctacgccaa      60

cagctccaac tacctctaga                                                   80

<210>  7
<211>  99

<212> DNA

<213> Artificial

<220>

<223> an artificially synthesized sequence

<400> 7

tccccggttc gaaaccgggc actacaaaaa ccaactttca aagaaagctg atgaggccga 60

aaggccgaaa cccattgggg taccccggat atcttttttt 99

<210> 8

<211> 26

<212> DNA

<213> Artificial

<220>

<223> an artificially syntheisized primer sequence

<400> 8

atgaaaagcc ctgctttgca acccct 26

<210> 9

<211> 24

<212>  DNA

<213>  Artificial

<220>

<223>  an artificially synthesized primer sequence

<400>  9

agttgcagtt tcagcattac tctt                                                24

<210>  10

<211>  19

<212>  DNA

<213>  Artificial

<220>

<223>  puromycin resistance gene

<400>  10

atgaccgagt acaagccca                                                      19

<210>  11

<211>  19

<212>  DNA

<213>  Artificial

<220>

<223>  puromycin resistance gene

<400>  11
ctcgccaccc gcgacgacg                                                    19

<210>  12
<211>  19
<212>  DNA
<213>  Artificial

<220>

<223>  puromycin resistance gene

<400>  12
caccgtcgac ccggaccgc                                                    19

<210>  13
<211>  19
<212>  DNA
<213>  Artificial

<220>

<223>  puromycin resistance gene

<400>  13
gaactcttcc tcacgcgcg                                                    19

<210>  14
<211>  19
<212>  DNA
<213>  Artificial

<220>
<223>  puromycin resistance gene

<400>  14
ggtgtgggtc gcggacgac                                                    19

<210>  15
<211>  19
<212>  DNA
<213>  Artificial

<220>
<223>  puromycin resistance gene

<400> 15
cagatggaag gcctcctgg 19

<210> 16
<211> 19
<212> DNA
<213> Artificial

<220>
<223> puromycin resistance gene

<400> 16
ggagcccgcg tggttcctg 19

<210> 17
<211> 19
<212> DNA
<213> Artificial

<220>
<223> puromycin resistance gene

<400> 17
gggtctgggc agcgccgtc 19

<210> 18

<211> 19

<212> DNA

<213> Artificial

<220>

<223> puromycin resistance gene

<400> 18

cctccccttc tacgagcgg 19

<210> 19

<211> 19

<212> DNA

<213> Artificial

<220>

<223> puromycin resistance gene

<400> 19

gcccggtgcc tgacgcccg 19

<210> 20

<211> 19

<212> DNA

<213> Homo sapiens

<220>

<221> misc_feature

<223> v-Ha-ras Harvey rat sarcoma viral oncogene homolog

<400> 20

atgacggaat ataagcttg 19

<210> 21

<211> 19

<212> DNA

<213> Homo sapiens

<220>

<221> misc_feature

<223> v-Ha-ras Harvey rat sarcoma viral oncogene homolog

<400> 21

gttggcgccg gcggtgtgg 19

<210> 22

<211> 19

<212> DNA

<213> Homo sapiens

<220>

<221> misc_feature

<223> v-Ha-ras Harvey rat sarcoma viral oncogene homolog

<400> 22

tacgacccca ctatagagg 19

<210> 23

<211> 19

<212> DNA

<213> Homo sapiens

<220>

<221> misc_feature

<223> v-Ha-ras Harvey rat sarcoma viral oncogene homolog

<400> 23

aggaggagta cagcgccat 19

<210> 24

<211> 19

<212> DNA

<213> Homo sapiens

<220>

<221> misc_feature

<223> v-Ha-ras Harvey rat sarcoma viral oncogene homolog

<400> 24

caacaccaag tcttttgag 19

<210> 25

<211> 19

<212> DNA

<213> Homo sapiens

<220>

<221> misc_feature

<223> v-Ha-ras Harvey rat sarcoma viral oncogene homolog

<400> 25

ggactcggat gacgtgccc 19

<210> 26

<211> 19

<212> DNA

<213> Homo sapiens

<220>

<221> misc_feature

<223> v-Ha-ras Harvey rat sarcoma viral oncogene homolog

<400> 26

tctcggcagg ctcaggacc 19

<210> 27

<211> 19

<212> DNA

<213> Homo sapiens

<220>

<221> misc_feature

<223> v-Ha-ras Harvey rat sarcoma viral oncogene homolog

<400> 27

gacccggcag ggagtggag 19

<210>  28

<211>  19

<212>  DNA

<213>  Homo sapiens

<220>

<221>  misc_feature

<223>  v-Ha-ras Harvey rat sarcoma viral oncogene homolog

<400>  28

gctgcggaag ctgaaccct                                                    19

<210>  29

<211>  19

<212>  DNA

<213>  Homo sapiens

<220>

<221>  misc_feature

<223>  v-Ha-ras Harvey rat sarcoma viral oncogene homolog

<400>  29

gtgtgtgctc tcctgagga                                                    19

<210> 30

<211> 10

<212> RNA

<213> Artificial

<220>

<223> an artificially synthesized sequence

<400> 30

cuuccuguca 10

<210> 31

<211> 29

<212> DNA

<213> Homo sapiens

<400> 31

aagugugcac cggcacagac augaagcug 29

<210> 32

<211> 29

<212> DNA

<213> Homo sapiens

<400> 32

acccaccugg acaugcuccg ccaccucua                                          29

<210> 33

<211> 29

<212> DNA

<213> Homo sapiens

<400> 33

ugcucaucgc ucacaaccaa gugaggcag                                          29

<210> 34

<211> 29

<212> DNA

<213> Homo sapiens

<400> 34

aagauccgga aguacacgau gcggagacu                                          29

<210> 35

<211> 29

<212> DNA

<213> Homo sapiens

<400> 35

gacacagcuu augcccuaug gcugccucu 29

<210> 36

<211> 29

<212> DNA

<213> Homo sapiens

<400> 36

ggcaagcuga cccugaaguu catctgcac 29

<210> 37

<211> 29

<212> DNA

<213> Artificial

<220>

<223> an artificially synthesized sequence

<400> 37

aagtgtgcac cggcacagac atgaagctg 29

<210> 38

<211> 42

<212> DNA

<213> Artificial

<220>

<223> an artificially synthesized operator sequence

<400> 38

tttaccactc cctatcagtg atagagaaaa gtgaaagtcg ag 42

<210> 39

<211> 24

<212> DNA

<213> Artificial

<220>

<223> an artificially synthesized primer sequence

<400> 39

atgtctgccc ctatgtctgc ccct 24

<210> 40

<211> 24

<212>  DNA

<213>  Artificial

<220>

<223>  an artificially synthesized primer sequence

<400>  40

acaacggtat ctctctgcag gcta                                              24

<210>  41

<211>  24

<212>  DNA

<213>  Artificial

<220>

<223>  an artificially synthesized primer sequence

<400>  41

atgatagatc ccgtcgtttt acaa                                              24

<210>  42

<211>  24

<212>  DNA

<213>  Artificial

<220>

<223>  an artificially synthesized primer sequence

<400>  42
gaaaatggtc tgctgctgct gaac                                              24

<210>  43
<211>  24
<212>  DNA
<213>  Artificial

<220>

<223>  an artificially synthesized primer sequence

<400>  43
atgtcgctcc acaaggtaaa cagt                                              24

<210>  44
<211>  24
<212>  DNA
<213>  Artificial

<220>

<223>  an artificially synthesized primer sequence

<400>  44
gatttttagt aaaccttgtt cagg                                              24

<210>  45
<211>  24
<212>  DNA
<213>  Artificial

<220>
<223>  an artificially synthesized primer sequence

<400>  45
tcgatatctg gtatacccaa gtgt                                              24

<210>  46
<211>  24
<212>  DNA
<213>  Artificial

<220>
<223>  an artificially synthesized primer sequence

<400> 46
ggttacgttg gtgtagatgg gcgc 24

<210> 47
<211> 24
<212> DNA
<213> Artificial

<220>
<223> an artificially synthesized primer sequence

<400> 47
cgctcatcgc cggtagccag cgcg 24

<210> 48
<211> 24
<212> DNA
<213> Artificial

<220>
<223> an artificially synthesized primer sequence

<400> 48
ccaatccaca tctgtgaaag aaag 24

<210> 49

<211> 21

<212> RNA

<213> Artificial

<220>

<223> an artificially synthesized siRNA sequence

<400> 49

cugcgugcac uucguggccg a 21

<210> 50

<211> 21

<212> RNA

<213> Artificial

<220>

<223> an artificially synthesized siRNA sequence

<400> 50

ggccacgaag ugcacgcagu u 21

<210> 51

<211> 21

<212> DNA

<213> Artificial

<220>

<223> an artificially synthesized primer sequence

<400> 51

ggccacgaag tgcacgcagt t 21

<210> 52

<211> 21

<212> DNA

<213> Artificial

<220>

<223> an artificially synthesized primer sequence

<400> 52

caccctggcc tgggtgtggg t 21

<210> 53

<211> 24

<212>  DNA

<213>  Artificial

<220>

<223>  an artificially synthesized primer sequence

<400>  53

tctggttgtg gaaagagtac cgca                                              24

<210>  54

<211>  25

<212>  DNA

<213>  Artificial

<220>

<223>  an artificially synthesized primer sequence

<400>  54

cctctttacg tagaatagag ccaag                                             25

<210>  55

<211>  5775

<212>  DNA

<213>  Homo sapiens

<400> 55

atgaaaagcc ctgctttgca acccctcagc atggcaggcc tgcagctcat gacccctgct 60

tcctcaccaa tgggtccttt ctttggactg ccatggcaac aagaagcaat tcatgataac 120

atttatacgc caagaaaata tcaggttgaa ctgcttgaag cagctctgga tcataatacc 180

atcgtctgtt taaacactgg ctcagggaag acatttattg ctagtactac tctactaaag 240

agctgtctct atctagatct aggggagact tcagctagaa atggaaaaag gacggtgttc 300

ttggtcaact ctgcaaacca ggttgctcaa caagtgtcag ctgtcagaac tcattcagat 360

ctcaaggttg gggaatactc aaacctagaa gtaaatgcat cttggacaaa agagagatgg 420

aaccaagagt ttactaagca ccaggttctc attatgactt gctatgtcgc cttgaatgtt 480

ttgaaaaatg gttacttatc actgtcagac attaaccttt tggtgtttga tgagtgtcat 540

cttgcaatcc tagaccaccc ctatcgagaa tttatgaagc tctgtgaaat ttgtccatca 600

tgtcctcgca ttttgggact aactgcttcc attttaaatg ggaaatggga tccagaggat 660

ttggaagaaa agtttcagaa actagagaaa attcttaaga gtaatgctga aactgcaact 720

gacctggtgg tcttagacag gtatacttct cagccatgtg agattgtggt ggattgtgga 780

ccatttactg acagaagtgg gctttatgaa agactgctga tggaattaga agaagcactt 840

aattttatca atgattgtaa tatatctgta cattcaaaag aaagagattc tactttaatt 900

tcgaaacaga tactatcaga ctgtcgtgcc gtattggtag ttctgggacc ctggtgtgca 960

gataaagtag ctggaatgat ggtaagagaa ctacagaaat acatcaaaca tgagcaagag 1020

gagctgcaca ggaaattttt attgtttaca gacactttcc taaggaaaat acatgcacta 1080

tgtgaagagc acttctcacc tgcctcactt gacctgaaat ttgtaactcc taaagtaatc 1140

aaactgctcg aaatcttacg caaatataaa ccatatgagc gacacagttt tgaaagcgtt 1200

gagtggtata ataatagaaa tcaggataat tatgtgtcat ggagtgattc tgaggatgat 1260

gatgaggatg aagaaattga agaaaaagag aagccagaga caaatttccc ttctcctttt 1320

accaacattt tgtgcggaat tatttttgtg gaaagaagat acacagcagt tgtcttaaac 1380

agattgataa aggaagctgg caaacaagat ccagagctgg cttatatcag tagcaatttc 1440

ataactggac atggcattgg gaagaatcag cctcgcaaca acacgatgga agcagaattc 1500

agaaaacagg aagaggtact taggaaattt cgagcacatg agaccaacct gcttattgca 1560

acaagtattg tagaagaggg tgttgatata ccaaaatgca acttggtggt tcgttttgat 1620

ttgcccacag aatatcgatc ctatgttcaa tctaaaggaa gagcaagggc acccatctct 1680

aattatataa tgttagcgga tacagacaaa ataaaaagtt ttgaagaaga ccttaaaacc 1740

tacaaagcta ttgaaaagat cttgagaaac aagtgttcca agtcggttga tactggtgag 1800

actgacattg atcctgtcat ggatgatgat cacgttttcc caccatatgt gttgaggcct 1860

gacgatggtg gtccacgagt cacaatcaac acggccattg gacacatcaa tagatactgt 1920

gctagattac caagtgatcc gtttactcat ctagctccta aatgcagaac ccgagagttg 1980

cctgatggta cattttattc aactctttat ctgccaatta actcacctct tcgagcctcc 2040

attgttggtc caccaatgag ctgtgtacga ttggctgaaa gagttgtcgc tctcatttgc 2100

tgtgagaaac tgcacaaaat tggcgaactg gatgaccatt tgatgccagt tgggaaagag 2160

actgttaaat atgaagagga gcttgatttg catgatgaag aagagaccag tgttccagga 2220

agaccaggtt ccacgaaacg aaggcagtgc tacccaaaag caattccaga gtgtttgagg 2280

gatagttatc ccagacctga tcagccctgt tacctgtatg tgataggaat ggttttaact 2340

acacctttac ctgatgaact caactttaga aggcggaagc tctatcctcc tgaagatacc 2400

acaagatgct ttggaatact gacggccaaa cccatacctc agattccaca ctttcctgtg 2460

tacacacgct ctggagaggt taccatatcc attgagttga agaagtctgg tttcatgttg 2520

tctctacaaa tgcttgagtt gattacaaga cttcaccagt atatattctc acatattctt 2580

cggcttgaaa aacctgcact agaatttaaa cctacagacg ctgattcagc atactgtgtt 2640

ctacctctta atgttgttaa tgactccagc actttggata ttgactttaa attcatggaa 2700

gatattgaga agtctgaagc tcgcataggc attcccagta caaagtatac aaaagaaaca 2760

ccctttgttt ttaaattaga agattaccaa gatgccgtta tcattccaag atatcgcaat 2820

tttgatcagc ctcatcgatt ttatgtagct gatgtgtaca ctgatcttac cccactcagt 2880

aaatttcctt cccctgagta tgaaactttt gcagaatatt ataaaacaaa gtacaacctt 2940

gacctaacca atctcaacca gccactgctg gatgtggacc acacatcttc aagacttaat 3000

cttttgacac ctcgacattt gaatcagaag gggaaagcgc ttcctttaag cagtgctgag 3060

aagaggaaag ccaaatggga aagtctgcag aataaacaga tactggttcc agaactctgt 3120

gctatacatc caattccagc atcactgtgg agaaaagctg tttgtctccc cagcatactt 3180

tatcgccttc actgcctttt gactgcagag gagctaagag cccagactgc cagcgatgct 3240

ggcgtgggag tcagatcact tcctgcggat tttagatacc ctaacttaga cttcgggtgg 3300

aaaaaatcta ttgacagcaa atctttcatc tcaatttcta actcctcttc agctgaaaat 3360

gataattact gtaagcacag cacaattgtc cctgaaaatg ctgcacatca aggtgctaat 3420

agaacctcct ctctagaaaa tcatgaccaa atgtctgtga actgcagaac gttgctcagc 3480

gagtcccctg gtaagctcca cgttgaagtt tcagcagatc ttacagcaat taatggtctt 3540

tcttacaatc aaaatctcgc caatggcagt tatgatttag ctaacagaga cttttgccaa 3600

ggaaatcagc taaattacta caagcaggaa atacccgtgc aaccaactac ctcatattcc 3660

attcagaatt tatacagtta cgagaaccag ccccagccca gcgatgaatg tactctcctg 3720

agtaataaat accttgatgg aaatgctaac aaatctacct cagatggaag tcctgtgatg 3780

gccgtaatgc ctggtacgac agacactatt caagtgctca agggcaggat ggattctgag 3840

cagagccctt ctattgggta ctcctcaagg actcttggcc ccaatcctgg acttattctt 3900

caggctttga ctctgtcaaa cgctagtgat ggatttaacc tggagcggct tgaaatgctt 3960

ggcgactcct ttttaaagca tgccatcacc acatatctat tttgcactta ccctgatgcg 4020

catgagggcc gcctttcata tatgagaagc aaaaaggtca gcaactgtaa tctgtatcgc 4080

cttggaaaaa agaagggact acccagccgc atggtggtgt caatatttga tccccctgtg 4140

aattggcttc ctcctggtta tgtagtaaat caagacaaaa gcaacacaga taaatgggaa 4200

aaagatgaaa tgacaaaaga ctgcatgctg gcgaatggca aactggatga ggattacgag 4260

gaggaggatg aggaggagga gagcctgatg tggagggctc cgaaggaaga ggctgactat 4320

gaagatgatt tcctggagta tgatcaggaa catatcagat ttatagataa tatgttaatg 4380

gggtcaggag cttttgtaaa gaaaatctct ctttctcctt tttcaaccac tgattctgca 4440

tatgaatgga aaatgcccaa aaaatcctcc ttaggtagta tgccattttc atcagatttt 4500

gaggattttg actacagctc ttgggatgca atgtgctatc tggatcctag caaagctgtt 4560

gaagaagatg actttgtggt ggggttctgg aatccatcag aagaaaactg tggtgttgac 4620

acgggaaagc agtccatttc ttacgacttg cacactgagc agtgtattgc tgacaaaagc 4680

atagcggact gtgtggaagc cctgctgggc tgctatttaa ccagctgtgg ggagagggct 4740

gctcagcttt tcctctgttc actggggctg aaggtgctcc cggtaattaa aaggactgat 4800

cgggaaaagg ccctgtgccc tactcgggag aatttcaaca gccaacaaaa gaacctttca 4860

gtgagctgtg ctgctgcttc tgtggccagt tcacgctctt ctgtattgaa agactcggaa 4920

tatggttgtt tgaagattcc accaagatgt atgtttgatc atccagatgc agataaaaca 4980

ctgaatcacc ttatatcggg gtttgaaaat tttgaaaaga aaatcaacta cagattcaag 5040

aataaggctt accttctcca ggcttttaca catgcctcct accactacaa tactatcact 5100

gattgttacc agcgcttaga attcctggga gatgcgattt tggactacct cataaccaag 5160

cacctttatg aagacccgcg gcagcactcc ccgggggtcc tgacagacct gcggtctgcc 5220

ctggtcaaca acaccatctt tgcatcgctg gctgtaaagt acgactacca caagtacttc 5280

aaagctgtct ctcctgagct cttccatgtc attgatgact ttgtgcagtt tcagcttgag 5340

aagaatgaaa tgcaaggaat ggattctgag cttaggagat ctgaggagga tgaagagaaa 5400

gaagaggata ttgaagttcc aaaggccatg ggggatattt ttgagtcgct tgctggtgcc 5460

atttacatgg atagtgggat gtcactggag acagtctggc aggtgtacta tcccatgatg 5520

cggccactaa tagaaaagtt ttctgcaaat gtaccccgtt cccctgtgcg agaattgctt 5580

gaaatggaac cagaaactgc caaatttagc ccggctgaga gaacttacga cgggaaggtc 5640

agagtcactg tggaagtagt aggaaagggg aaatttaaag gtgttggtcg aagttacagg 5700

attgccaaat ctgcagcagc aagaagagcc ctccgaagcc tcaaagctaa tcaacctcag 5760

gttcccaata gctga 5775

<210> 56

<211> 1924

<212> PRT

<213> Homo sapiens

<400> 56